# EUROPÄISCHE PATENTSCHRIFT

(11) **EP 1 194 416 B1**
(45) Veröffentlichungstag und Bekanntmachung des Hinweises auf die Patenterteilung: **08.09.2004**
(21) Anmeldenummer: 00936861.4
(22) Anmeldetag: 06.06.2000
(51) Int. Cl.: C07D 239/52, C07D 239/34, C07D 405/12, A01N 43/54

(54) **HALOGENPYRIMIDINE**
HALOPYRIMIDINES
PYRIMIDINES HALOGENEES

(30) Priorität: 18.06.1999 DE 19927914; 04.02.2000 DE 10005039
(43) Veröffentlichungstag der Anmeldung: 10.04.2002
(73) Patentinhaber: Bayer CropScience AG, 40789 Monheim (DE)
(72) Erfinder: KRÜGER, Bernd-Wieland, D-51467 Bergisch Gladbach (DE); GAYER, Herbert, D-40789 Monheim (DE); GERDES, Peter, D-52080 Aachen (DE); HEINEMANN, Ulrich, D-42799 Leichlingen (DE); MAULER-MACHNIK, Astrid, D-42799 Leichlingen (DE); VAUPEL, Martin, D-42799 Leichlingen (DE); MAURER, Fritz, D-40789 Monheim (DE); WACHENDORFF-NEUMANN, Ulrike, D-56566 Neuwied (DE); HÄNSSLER, Gerd, D-51381 Leverkusen (DE); KUCK, Karl-Heinz, D-40764 Langenfeld (DE); LÖSEL, Peter, D-40789 Monheim (DE); DUNKEL, Ralf, D-40789 Monheim (DE)
(86) Internationale Anmeldenummer: PCT/EP2000/005160
(87) Internationale Veröffentlichungsnummer: WO 2000/078732

(56) Entgegenhaltungen:
- EP-A- 0 468 684
- DE-A- 19 646 407
- DE-A- 19 723 195
- GB-A- 2 253 624

## Beschreibung

Die Erfindung betrifft neue Halogenpyrimidine, mehrere Verfahren zu ihrer Herstellung und ihre Verwendung als Schädlingsbekämpfungsmittel.

Bestimmte Pyrimidine mit ähnlichem Substitutionsmuster, sowie deren fungizide Wirkung sind bereits bekannt geworden (GB-A 2253624). Die Wirkung dieser vorbekannten Verbindungen ist jedoch insbesondere bei niedrigen Aufwandmengen und Konzentrationen nicht in allen Anwendungsgebieten völlig zufriedenstellend.

Es wurden nun die neuen Halogenpyrimidine der allgemeinen Formel (I) gefunden, 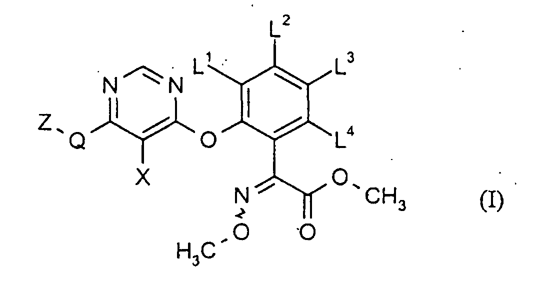 in welcher
- Z: für jeweils substituiertes oder unsubstituiertes Cycloalkyl, Aryl oder Heterocyclyl steht,
- Q: für Sauerstoff oder Schwefel steht,
- x: für Halogen steht und
- L¹, L², L³ und L⁴: gleich oder verschieden sind und unabhängig voneinander jeweils für Wasserstoff, Halogen, Cyano, Nitro, jeweils gegebenenfalls durch Halogen substituiertes Alkyl, Alkoxy, Alkylthio, Alkylsulfinyl oder Alkylsulfonyl stehen.

In EP-A2-0 468 684 werden Propensäurederivate, ein Verfahren zu ihrer Herstellung und ihre Verwendung als Fungizide beschrieben. Aus DE 197 23 195 sind Fluormethoximinoverbindungen bekannt. In DE 196 46 407 werden Halogenpyrimidine beschrieben.

In den Definitionen sind die gesättigten oder ungesättigten Kohlenwasserstoffketten, wie Alkyl, Alkandiyl, Alkenyl oder Alkinyl, auch in Verknüpfung mit Heteroatomen, wie beispielsweise in Alkoxy, Alkylthio oder Alkylamino, jeweils geradkettig oder verzweigt. Bevorzugt sind, wenn nicht anders angegeben, Kohlenwasserstoffketten mit 1 bis 6 Kohlenstoffatomen.

Halogen steht im allgemeinen für Fluor, Chlor, Brom oder Iod, vorzugsweise für Fluor, Chlor oder Brom, insbesondere für Fluor oder Chlor.

Aryl steht für aromatische, mono oder polycyclische Kohlenwasserstoffringe, wie z.B. Phenyl, Naphthyl, Anthranyl, Phenanthryl, vorzugsweise Phenyl oder Naphthyl, insbesondere Phenyl.

Heterocyclyl steht für gesättigte oder ungesättigte, sowie aromatische, ringförmige Verbindungen, in denen mindestens ein Ringglied ein Heteroatom, d. h. ein von Kohlenstoff verschiedenes Atom, ist. Enthält der Ring mehrere Heteroatome, können diese gleich oder verschieden sein. Heteroatome sind bevorzugt Sauerstoff, Stickstoff oder Schwefel. Enthält der Ring mehrere Sauerstoffatome, stehen diese nicht benachbart. Gegebenenfalls bilden die ringförmigen Verbindungen mit weiteren carbocyclischen oder heterocyclischen, ankondensierten oder überbrückten Ringen gemeinsam ein polycyclisches Ringsystem. Bevorzugt sind mono- oder bicyclische Ringsysteme, insbesondere mono- oder bicyclische, aromatische Ringsysteme.

Cycloalkyl steht für gesättigte, carbocyclische, ringförmige Verbindungen, die gegebenenfalls mit weiteren carbocyclischen, ankondensierten oder überbrückten Ringen ein polycyclisches Ringsystem bilden.

Ein polycyclisches Ringsystem kann mit einem heterocyclischen Ring oder einem ankondensierten carbocyclischen Ring verknüpft sein. Das so beschriebene Heterocyclyl kann auch einfach oder mehrfach substituiert sein, vorzugsweise durch Methyl, Ethyl, Halogen oder Chlor. Bevorzugt sind mono- oder bicyclische Ringsysteme, insbesondere mono- oder bicyclische Ringsysteme.

Halogenalkoxy steht für teilweise oder vollständig halogeniertes Alkoxy. Bei mehrfach halogeniertem Halogenalkoxy können die Halogenatome gleich oder verschieden sein. Bevorzugte Halogenatome sind Fluor und insbesondere Chlor. Trägt das Halogenalkoxy noch weitere Substituenten, reduziert sich die maximal mögliche Zahl der Halogenatome auf die verschiedenen freien Valenzen. Bevorzugt sind, wenn nicht anders angegeben, Kohlenwasserstoffketten mit 1 bis 6 Kohlenstoffatomen.

Halogenalkyl steht für teilweise oder vollständig halogeniertes Alkyl. Bei mehrfach halogeniertem Halogenalkyl können die Halogenatome gleich oder verschieden sein. Bevorzugte Halogenatome sind Fluor und Chlor, insbesondere Fluor. Trägt das Halogenalkyl noch weitere Substituenten, reduziert sich die maximal mögliche Zahl der Halogenatome auf die verbleibenden freien Valenzen. Bevorzugt sind, wenn nicht anders angegeben, Kohlenwasserstoffketten mit 1 bis 6 Kohlenstoffatomen.

Weiterhin wurde gefunden, dass man die neuen Halogenpyrimidine der allgemeinen Formel (I) erhält, wenn man
a) 2-(2-Hydroxy-phenyl)-2-methoxyiminoessigester der Formel (II), 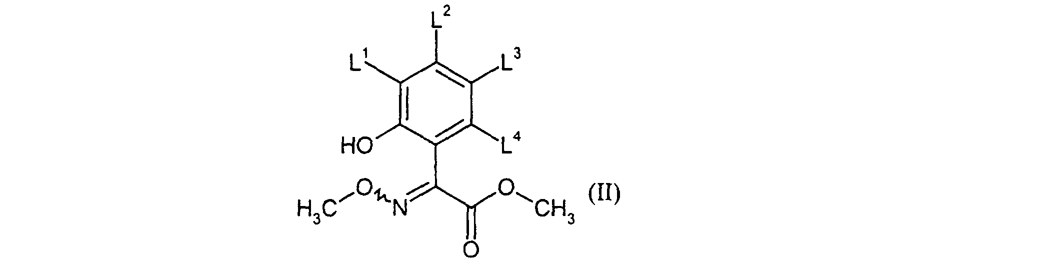 in welcher
   - L¹, L², L³ und L⁴: die oben angegebenen Bedeutungen haben,
   mit einem substituierten Halogenpyrimidin der allgemeinen Formel (III), 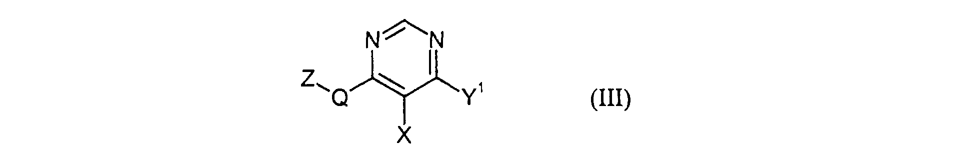 in welcher
   - Z, Q und X: die oben angegebenen Bedeutungen haben und
   - Y¹: für Halogen steht,
   gegebenenfalls in Gegenwart eines Verdünnungsmittels, gegebenenfalls in Gegenwart eines Säureakzeptors und gegebenenfalls in Gegenwart eines Katalysators, umsetzt, oder wenn man
b) Phenoxypyrimidine der allgemeinen Formel (IV) 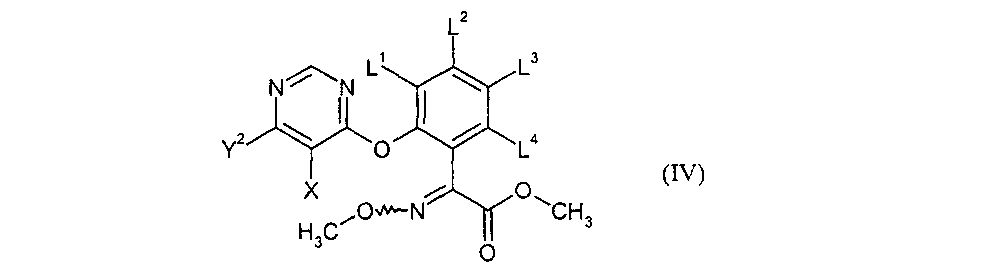
in welcher
- X, L¹, L², L³ und L⁴: die oben angegebenen Bedeutungen haben und
- Y²: für Halogen steht,
mit einer Ringverbindung der allgemeinen Formel (V),

Z-Q-H (V)

in welcher
- Z und Q: die oben angegebenen Bedeutungen haben,
gegebenenfalls in Gegenwart eines Verdünnungsmittels, gegebenenfalls in Gegenwart eines Säureakzeptors und gegebenenfalls in Gegenwart eines Katalysators, umsetzt.

Schließlich wurde gefunden, dass die neuen Halogenpyrimidine der allgemeinen Formel (I) eine sehr starke Wirkung gegen Pflanzenschädlinge zeigen.

Die erfindungsgemäßen Verbindungen können gegebenenfalls als Mischungen verschiedener möglicher isomerer Formen, insbesondere von Stereoisomeren, wie z. B. E- und Z-, vorliegen. Es werden sowohl die E- als auch die Z-Isomeren, wie auch beliebige Mischungen dieser Isomeren, beansprucht.

Gegenstand der Erfindung sind vorzugsweise Verbindungen der Formel (I), in welcher
- Z: für jeweils gegebenenfalls einfach bis zweifach durch Halogen, Alkyl, oder Hydroxy substituiertes Cycloalkyl mit 3 bis 7 Kohlenstoffatomen;
für gegebenenfalls durch Alkyl mit 1 bis 4 Kohlenstoffatomen oder Halogen substituiertes Heterocyclyl mit 3 bis 7 Ringgliedem;
oder für jeweils gegebenenfalls einfach bis vierfach, gleich oder verschieden substituiertes Phenyl oder Naphthyl steht, wobei die möglichen Substituenten vorzugsweise aus der nachstehenden Aufzählung ausgewählt sind:
Halogen, Cyano, Nitro, Amino, Hydroxy, Formyl, Carboxy, Carbamoyl, Thiocarbamoyl;
jeweils geradkettiges oder verzweigtes Alkyl, Hydroxyalkyl, Oxoalkyl, Alkoxy, Alkoxyalkyl, Alkylthioalkyl, Dialkoxyalkyl, Mercaptoalkyl, Alkylthio, Alkylsulfinyl oder Alkylsulfonyl mit jeweils 1 bis 8 Kohlenstoffatomen;
jeweils geradkettiges oder verzweigtes Alkenyl oder Alkenyloxy mit jeweils 2 bis 6 Kohlenstoffatomen;
jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen;
jeweils geradkettiges oder verzweigtes Halogenalkenyl oder Halogenalkenyloxy mit jeweils 2 bis 6 Kohlenstoffatomen und 1 bis 11 gleichen oder verschiedenen Halogenatomen;
jeweils geradkettiges oder verzweigtes Alkylamino, Dialkylamino,
Alkylcarbonyl, Alkylcarbonyloxy, Alkoxycarbonyl, Alkylaminocarbonyl, Dialkylaminocarbonyl, Arylalkylaminocarbonyl, Dialkylaminocarbonyloxy, Alkenylcarbonyl oder Alkinylcarbonyl, mit 1 bis 6 Kohlenstoffatomen in den jeweiligen Kohlenwasserstoffketten;
Cycloalkyl oder Cycloalkyloxy mit jeweils 3 bis 6 Kohlenstoffatomen;
jeweils gegebenenfalls einfach bis vierfach, gleich oder verschieden durch Fluor, Chlor, Oxo, Methyl, Trifluormethyl oder Ethyl substituiertes, jeweils zweifach verknüpftes Alkylen mit 3 oder 4 Kohlenstoffatomen, Oxyalkylen mit 2 oder 3 Kohlenstoffatomen oder Dioxyalkylen mit 1 oder 2 Kohlenstoffatomen; oder eine Gruppierung 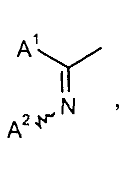 worin
A¹ für Wasserstoff, Hydroxy oder Alkyl mit 1 bis 4 Kohlenstoffatomen oder Cycloalkyl mit 1 bis 6 Kohlenstoffatomen steht und
A² für Hydroxy, Amino, Methylamino, Phenyl, Benzyl oder für jeweils gegebenenfalls durch Cyano, Hydroxy, Alkoxy, Alkylthio, Alkylamino, Dialkylamino oder Phenyl substituiertes Alkyl oder Alkoxy mit 1 bis 4 Kohlenstoffatomen, oder für Alkenyloxy oder Alkinyloxy mit jeweils 2 bis 4 Kohlenstoffatomen steht, sowie jeweils gegebenenfalls im Ringteil einfach bis dreifach durch Halogen, und/oder geradkettiges oder verzweigtes Alkyl oder Alkoxy mit 1 bis 4 Kohlenstoffatomen substituiertes Phenyl, Phenoxy, Phenyllthio, Benzoyl, Benzoylethenyl, Cinnamoyl, Heterocyclyl oder Phenylalkyl, Phenylalkyloxy, Phenylalkylthio, oder Heterocyclylalkyl, mit jeweils 1 bis 3 Kohlenstoffatomen in den jeweiligen Alkylteilen,
- Q: für Sauerstoff oder Schwefel steht,
- X: für Fluor, Chlor oder Brom steht und
- L¹, L², L³ und L⁴: gleich oder verschieden sind und unabhängig voneinander jeweils für Wasserstoff, Halogen, Cyano, Nitro, jeweils gegebenenfalls durch 1 bis 5 Halogenatome substituiertes Alkyl, Alkoxy, Alkylthio, Alkylsulfinyl oder Alkylsulfonyl mit jeweils 1 bis 6 Kohlenstoffatomen stehen.

Die Erfindung betrifft insbesondere Verbindungen der Formel (I), in welcher
- Z: für jeweils gegebenenfalls einfach bis zweifach durch Fluor, Chlor, Methyl, Ethyl oder Hydroxy substituiertes Cyclopentyl oder Cyclohexyl;
für gegebenenfalls durch Methyl, Ethyl oder Chlor substituiertes Thienyl, Pyridyl oder Furyl;
oder für jeweils gegebenenfalls einfach bis vierfach, gleich oder verschieden substituiertes Phenyl oder Naphthyl steht, wobei die möglichen Substituenten vorzugsweise aus der nachstehenden Aufzählung ausgewählt sind:
Fluor, Chlor, Brom, Iod, Cyano, Nitro, Amino, Hydroxy, Formyl, Carboxy, Carbamoyl, Thiocarbamoyl,
Methyl, Ethyl, n- oder i-Propyl, n-, i-, s- oder t-Butyl, 1-, 2-, 3-, neo-Pentyl, 1-, 2-, 3-, 4-(2-Methylbutyl), 1-, 2-, 3-Hexyl, 1-, 2-, 3-, 4-, 5-(2-Methylpentyl), 1-, 2-, 3-(3-Methylpentyl), 2-Ethylbutyl, 1-, 3-, 4-(2,2-Dimetylbutyl), 1-, 2-(2,3-Dimethylbutyl), Hydroxymethyl, Hydroxyethyl, 3-Oxobutyl, Methoxymethyl, Dimethoxymethyl,
Methoxy, Ethoxy, n- oder i-Propoxy, Methoxymethyl, Ethoxymethyl,
Mercaptomethyl, Methylthio, Ethylthio, n- oder i-Propylthio, Methylsulfinyl, Ethylsulfinyl, Methylsulfonyl oder Ethylsulfonyl, Methylthiomethyl, Ethylthiomethyl,
Vinyl, Allyl, 2-Methylallyl, Propen-1-yl, Crotonyl, Propargyl, Vinyloxy, Allyloxy, 2-Methylallyloxy, Propen-1-yloxy, Crotonyloxy, Propargyloxy;
Trifluormethyl, Trifluorethyl,
Difluormethoxy, Trifluormethoxy, Difluorchlormethoxy, Trifluorethoxy, Pentafluorpropoxy, Difluormethylthio, Trifluormethylthio, Difluorchlormethylthio, Trifluormethylsulfinyl oder Trifluormethylsulfonyl,
Methylamino, Ethylamino, n- oder i-Propylamino, Dimethylamino, Diethylamino,
Acetyl, Propionyl, Methoxycarbonyl, Ethoxycarbonyl, Methylaminocarbonyl, Ethylaminocarbonyl, Dimethylaminocarbonyl, Diethylaminocarbonyl, Dimethylaminocarbonyloxy, Diethylaminocarbonyloxy, Benzylaminocarbonyl, Acryloyl, Propioloyl,
Cyclopentyl, Cyclohexyl,
jeweils gegebenenfalls einfach bis vierfach, gleich oder verschieden durch Fluor, Chlor, Oxo, Methyl oder Trifluormethyl substituiertes, jeweils zweifach verknüpftes Propandiyl, Ethylenoxy, Methylendioxy, Ethylendioxy oder eine Gruppierung 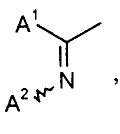 wobei
A¹ für Wasserstoff, Methyl oder Hydroxy steht und
A² für Hydroxy, Methoxy, Ethoxy, Amino, Methylamino, Phenyl, Benzyl oder Hydroxyethyl steht, sowie jeweils gegebenenfalls im Ringteil einfach bis vierfach durch Halogen, und/oder geradkettiges oder verzweigtes Alkyl oder Alkoxy mit 1 bis 4 Kohlenstoffatomen substituiertes Phenyl, Phenoxy, Phenyllthio, Benzoyl, Benzoylethenyl, Cinnamoyl, Benzyl, Phenylethyl, Phenylpropyl, Benzyloxy, Benzylthio, 5,6-Dihydro-1,4,2-dioxazin-3-ylmethyl, Triazolylmethyl, Benzoxazol-2-ylmethyl, 1,3-Dioxan-2-yl, Benzimidazol-2-yl, Dioxol-2-yl, Oxadiazolyl, 2,3-Dihydro-1,4-benzodioxin-6-yl, Benzodioxol-4-yl,
- Q: für Sauerstoff steht,
- X: für Fluor steht und
- L¹, L², L³ und L⁴: gleich oder verschieden sind und unabhängig voneinander jeweils für Wasserstoff, Methyl, Ethyl, n- oder i-Propyl, n-, i-, s- oder t-Butyl stehen.

In einer bevorzugten Gruppe von Verbindungen der Formel (I) steht Z für gegebenenfalls substituiertes Phenyl, Naphthyl oder Pyridyl.

In einer ganz besonders bevorzugten Gruppen von Verbindungen der Formel (I) steht Z für substituiertes Phenyl, wobei die möglichen Substituenten vorzugsweise die in den voranstehenden Vorzugsbereichen genannt sind.

Besonders bevorzugt sind Verbindungen der Formel (I), in denen Q für Sauerstoff steht.

Besonders bevorzugt sind Verbindungen der Formel (I), in denen Z für gegebenenfalls substituiertes Phenyl steht, wobei die Substituenten vorzugsweise aus der nachstehenden Aufzählung ausgewählt sind: Halogen, Cyano, jeweils geradkettiges oder verzweigtes Alkyl, Alkylthio, Alkylthioalkyl, Halogenalkyl, Halogenthioalkyl.

Besonders bevorzugt sind Verbindungen der Formel (I), in denen X für Fluor steht.

In einer weiteren ganz besonders bevorzugten Gruppe von Verbindungen stehen
- L¹, L² und L³: für Wasserstoff und
- L⁴: für Wasserstoff oder für Methyl.

Die oben aufgeführten allgemeinen oder in Vorzugsbereichen angegebenen Restedefinitionen gelten sowohl für die Endprodukte der Formel (I) als auch entsprechend für die jeweils zur Herstellung benötigten Ausgangsstoffe bzw. Zwischenprodukte.

Die in den jeweiligen Kombinationen bzw. bevorzugten Kombinationen von Resten im einzelnen für diese Reste angegebenen Restedefinitionen werden unabhängig von der jeweilig angegebenen Kombination, beliebig auch durch Restedefinitionen anderer Vorzugsbereiche ersetzt.

Die zur Durchführung des erfindungsgemäßen Verfahrens a) als Ausgangsstoffe benötigten 2-(2-Hydroxy-phenyl)-2-methoxyiminoessigester sind durch die Formel (II) allgemein definiert. In dieser Formel (II) haben L¹, L², L³ und L⁴ vorzugsweise bzw. insbesondere diejenigen Bedeutungen, die bereits im Zusammenhang mit der Beschreibung der erfindungsgemäßen Verbindungen der Formel (I) als bevorzugt bzw. als insbesondere bevorzugt für L¹, L², L³ und L⁴ angegeben wurden.

Die Ausgangsstoffe der Formel (II) sind bekannt und können nach bekannten Verfahren hergestellt werden (vergleiche z. B. WO-A 94-05626, GB-A 2249092)

Die zur Durchführung des erfindungsgemäßen Verfahrens a) weiterhin als Ausgangsstoffe benötigten Halogenpyrimidine sind durch die Formel (III) allgemein definiert. In dieser Formel (III) haben Z, Q und X vorzugsweise bzw. insbesondere diejenigen Bedeutungen, die bereits im Zusammenhang mit der Beschreibung der erfindungsgemäßen Verbindungen der Formel (I) als bevorzugt bzw. als insbesondere bevorzugt für Z, Q und X angegeben wurden. Y¹ steht für Halogen, vorzugsweise für Fluor oder Chlor.

Die Ausgangsstoffe der Formel (III) sind bekannt und/oder können nach bekannten Methoden hergestellt werden (vergleiche z. B. DE-A 4340181; Chem.Ber., 90 <1957> 942, 951).

Die zur Durchführung des erfindungsgemäßen Verfahrens b) als Ausgangsstoffe benötigten Phenoxypyrimidine sind durch die Formel (IV) allgemein definiert. In dieser Formel (IV) haben X, L¹, L², L³ und L⁴ vorzugsweise bzw. insbesondere diejenigen Bedeutungen, die bereits im Zusammenhang mit der Beschreibung der erfindungsgemäßen Verbindungen der Formel (I) als bevorzugt bzw. als insbesondere bevorzugt für X, L¹, L², L³ und L⁴ angegeben wurden. Y² steht für Halogen, vorzugsweise für Fluor oder Chlor.

Die Ausgangsstoffe der Formel (IV) sind neu und ebenfalls Gegenstand der vorliegenden Anmeldung. Sie sind wichtige Zwischenprodukte beispielhaft und vorzugsweise zur Herstellung von Schädlingsbekämpfungsmitteln.

Die Phenoxypyrimidine der allgemeinen Formel (IV) werden erhalten (Verfahren b-1), wenn man 2-(2-Hydroxy-phenyl)-2-methoxyimino-essigester der Formel (II) mit einem Trihalogenpyrimidin der allgemeinen Formel (VI) 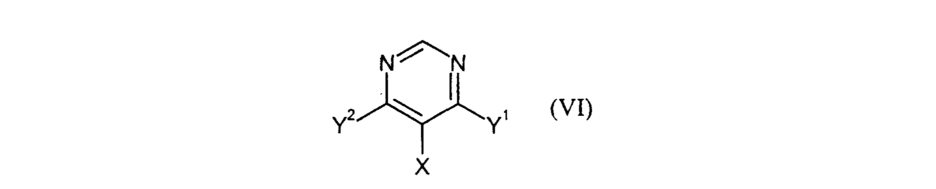 in welcher
- X, Y¹ und Y²: gleich oder verschieden sind und jeweils für Halogen stehen,
gegebenenfalls in Gegenwart eines Verdünnungsmittels, gegebenenfalls in Gegenwart eines Säureakzeptors und gegebenenfalls in Gegenwart eines Katalysators, umsetzt.

Die zur Durchführung des erfindungsgemäßen Verfahrens b-1) als Ausgangsstoffe benötigten Hydroxyverbindungen der Formel (II) sind bereits im Zusammenhang mit der Beschreibung des erfindungsgemäßen Verfahrens a) beschrieben worden.

Die weiterhin zur Durchführung des erfindungsgemäßen Verfahrens b-1) als Ausgangsstoffe benötigten Trihalogenpyrimidine sind durch die Formel (VI) allgemein definiert. In dieser Formel (VI) stehen X, Y¹ und Y² für Halogen, vorzugsweise für Fluor oder Chlor.

Die Trihalogenpyrimidine sind bekannt und/oder können nach bekannten Methoden hergestellt werden (vergleiche z.B. Chesterfield et al., J. Chem. Soc., 1955; 3478, 3480; WO-A 97-27189).

Die weiterhin zur Durchführung des erfindungsgemäßen Verfahrens b) als Ausgangsstoffe benötigten Ringverbindungen sind durch die Formel (V) allgemein definiert. In dieser Formel (V) haben Z und Q vorzugsweise bzw. insbesondere diejenigen Bedeutungen, die bereits im Zusammenhang mit der Beschreibung der erfindungsgemäßen Verbindungen der Formel (I) als bevorzugt bzw. als insbesondere bevorzugt für Z und Q angegeben wurden.

Die Ringverbindungen der Formel (V) sind bekannte Synthesechemikalien oder können nach einfachen Methoden hergestellt werden.

Als Verdünnungsmittel zur Durchführung der erfindungsgemäßen Verfahren a), b) und b-1) kommen alle inerten organischen Lösungsmittel in Betracht. Hierzu gehören beispielhaft und vorzugsweise Ether, wie beispielsweise Diethylether, Diisopropylether, Methyl-t-butylether, Methyl-t-Amylether, Dioxan, Tetrahydrofuran, 1,2-Dimethoxyethan, 1,2-Diethoxyethan oder Anisol; Nitrile, wie beispielsweise Acetonitril, Propionitril, n- oder i-Butyronitril oder Benzonitril; Amide, wie beispielsweise N,N-Dimethylformamid, N,N-Dimethylacetamid, N-Methylformanilid, N-Methylpyrrolidon oder Hexamethylphosphorsäuretriamid; Sulfoxide, wie beispielsweise Dimethylsulfoxid; oder Sulfone, wie beispielsweise Sulfolan.

Die erfindungsgemäßen Verfahren a), b) und b-1) werden gegebenenfalls in Gegenwart eines geeigneten Säureakzeptors durchgeführt. Als solche kommen alle üblichen anorganischen oder organischen Basen infrage. Hierzu gehören beispielhaft und vorzugsweise Erdalkalimetall- oder Alkalimetallhydride, -hydroxide, -alkoholate, - carbonate oder -hydrogencarbonate, wie beispielsweise Natriumhydrid, Natriumamid, Kalium-tert.-butylat, Natriumhydroxid, Kaliumhydroxid, Natriumcarbonat, Kaliumcarbonat, Kaliumhydrogencarbonat oder Natriumhydrogencarbonat.

Als Katalysatoren für die erfindungsgemäßen Verfahren a), b) und b-1) eignen sich alle Kupfer(I)-Salze, wie beispielsweise Kupfer(I)-chlorid, Kupfer(I)-bromid oder Kupfer(I)-iodid.

Die Reaktionstemperaturen können bei der Durchführung der erfindungsgemäßen Verfahren a), b) und b-1) in einem größeren Bereich variiert werden. Im allgemeinen arbeitet man bei Temperaturen von -20°C bis 100°C, vorzugsweise bei Temperaturen von -10°C bis 80°C.

Zur Durchführung des erfindungsgemäßen Verfahrens a) zur Herstellung der Verbindungen der Formel (I) setzt man pro Mol des 2-(2-Hydroxy-phenyl)-2-methoxyimino- essigesters der Formel (II) im allgemeinen 0,5 bis 15 mol, vorzugsweise 0,8 bis 8 mol substituiertes Halogenpyrimidin der Formel (III) ein.

Zur Durchführung des erfindungsgemäßen Verfahrens b) zur Herstellung der Verbindungen der Formel (I) setzt man pro Mol des Phenoxypyrimidins der Formel (IV) im allgemeinen 0,5 bis 15 mol, vorzugsweise 0,8 bis 8 mol einer Ringverbindung der allgemeinen Formel (V) ein.

Zur Durchführung des erfindungsgemäßen Verfahrens b-1) zur Herstellung der Verbindungen der Formel (IV) setzt man pro Mol des 2-(2-Hydroxy-phenyl)-2-methoxyimino-essigesters der Formel (II) im allgemeinen 1 bis 15 mol, vorzugsweise 2 bis 8 mol eines Trihalogenpyrimidins der allgemeinen Formel (VI) ein.

Alle erfindungsgemäßen Verfahren werden im allgemeinen unter Normaldruck durchgeführt. Es ist jedoch auch möglich, unter erhöhtem oder vermindertem Druck - im allgemeinen zwischen 0,1 bar und 10 bar - zu arbeiten.

Die Reaktionsdurchführung, Aufarbeitung und Isolierung der Reaktionsprodukte erfolgt nach allgemein üblichen Verfahren (vergleiche auch die Herstellungsbeispiele).

### Biologie

Die erfindungsgemäßen Stoffe weisen eine starke mikrobizide Wirkung auf und können zur Bekämpfung von unerwünschten Mikroorganismen, wie Fungi und Bakterien, im Pflanzenschutz und im Materialschutz eingesetzt werden.

Fungizide lassen sich Pflanzenschutz zur Bekämpfung von Plasmodiophoromycetes, Oomycetes, Chytridiomycetes, Zygomycetes, Ascomycetes, Basidiomycetes und Deuteromycetes einsetzen.

Bakterizide lassen sich im Pflanzenschutz zur Bekämpfung von Pseudomonadaceae, Rhizobiaceae, Enterobacteriaceae, Corynebacteriaceae und Streptomycetaceae einsetzen.

Beispielhaft aber nicht begrenzend seien einige Erreger von pilzlichen und bakteriellen Erkrankungen, die unter die oben aufgezählten Oberbegriffe fallen, genannt:
Xanthomonas-Arten, wie beispielsweise Xanthomonas campestris pv. oryzae;
Pseudomonas-Arten, wie beispielsweise Pseudomonas syringae pv. lachrymans;
Erwinia-Arten, wie beispielsweise Erwinia amylovora;
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Bremia-Arten, wie beispielsweise Bremia lactucae;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea
(Konidienform: Drechslera, Syn: Helminthosporium);
Cochliobolus-Arten, wie beispielsweise Cochliobolus sativus (Konidienform: Drechslera, Syn: Helminthosporium);
Uromyces-Arten, wie beispielsweise Uromyces appendiculatus;
Puccinia-Arten, wie beispielsweise Puccinia recondita;
Sclerotinia-Arten, wie beispielsweise Sclerotinia sclerotiorum;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Sproß, blatt, Blüte und Wurzel verstanden werden, wobei beispielhaft Blätter, Nadeln, Stengel, Stämme, Blüten, Fruchtkörper, Früchte und Samen sowie Wurzeln, Knollen und Rhizome aufgeführt werden. Zu den Pflanzenteilen gehört auch Erntegut sowie vegatives und generatives Vermehrungsmaterial, beispielsweise Stecklinge, Knollen, Rhizome, Ableger und Samen.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen und bei Vermehrungsmaterial, insbesondere bei Samen, weiterhin durch ein- oder mehrschichtiges Umhüllen.

Dabei lassen sich die erfindungsgemäßen Wirkstoffe mit besonders gutem Erfolg zur Bekämpfung von Getreidekrankheiten, wie beispielsweise gegen Leptosphaeria-Arten, von Krankheiten im Wein-, Obst- und Gemüseanbau, wie beispielsweise gegen Venturia-, Sphaerotheca-, Phytophtora- und Plasmopara-Arten, oder von Reiskrankheiten, wie beispielsweise gegen Pyricularia-Arten, einsetzen.

Die erfindungsgemäßen Wirkstoffe eignen sich auch zur Steigerung des Ernteertrages. Sie sind außerdem mindertoxisch und weisen eine gute Pflanzenverträglichkeit auf.

Die erfindungsgemäßen Wirkstoffe können gegebenenfalls in bestimmten Konzentrationen und Aufwandmengen auch als Herbizide, zur Beeinflussung des Pflanzenwachstums, sowie zur Bekämpfung von tierischen Schädlingen verwendet werden. Sie lassen sich gegebenenfalls auch als Zwischen- und Vorprodukte für die Synthese weiterer Wirkstoffe einsetzen.

Die Wirkstoffe können in Abhängigkeit von ihren jeweiligen physikalischen und/oder chemischen Eigenschaften in die üblichen Formulierungen überführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycolether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, kömige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Auch eine Mischung mit anderen bekannten Wirkstoffen, wie Herbiziden oder mit Düngemitteln und Wachstumsregulatoren ist möglich.

Darüber hinaus weisen die erfindungsgemäßen Verbindungen der Formel (I) auch sehr gute antimykotische Wirkungen auf. Sie besitzen ein sehr breites antimykotisches Wirkungsspektrum, insbesondere gegen Dermatophyten und Sproßpilze, Schimmel und diphasische Pilze (z.B. gegen Candida-Spezies wie Candida albicans, Candida glabrata) wie Epidermophyton floccosum, Aspergillus-Spezies wie Aspergillus niger und Aspergillus fumigatus, Trichophyton-Spezies wie Trichophyton mentagrophytes, Microsporon-Spezies wie Microsporon canis und audouinii. Die Aufzählung dieser Pilze stellt keinesfalls eine Beschränkung des erfaßbaren mykotischen Spektrums dar, sondern hat nur erläuternden Charakter.

Die Wirkstoffe können als solche, in Form ihrer Formulierungen oder den daraus bereiteten Anwendungsformen, wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Beim Einsatz der erfindungsgemäßen Wirkstoffe als Fungizide können die Aufwandmengen je nach Applikationsart innerhalb eines größeren Bereiches variiert werden. Bei der Behandlung von Pflanzenteilen liegen die Aufwandmengen an Wirkstoff im allgemeinen zwischen 0,1 und 10.000 g/ha, vorzugsweise zwischen 10 und 1.000 g/ha. Bei der Saatgutbehandlung liegen die Aufwandmengen an Wirkstoff im allgemeinen zwischen 0,001 und 50 g pro Kilogramm Saatgut, vorzugsweise zwischen 0,01 und 10 g pro Kilogramm Saatgut. Bei der Behandlung des Bodens liegen die Aufwandmengen an Wirkstoff im allgemeinen zwischen 0,1 und 10.000 g/ha, vorzugsweise zwischen 1 und 5.000 g/ha.

Die Wirkstoffe eignen sich bei guter Pflanzenverträglichkeit und günstiger Warmblütertoxizität zur Bekämpfung von tierischen Schädlingen, insbesondere Insekten, Spinnentieren und Nematoden, die in der Landwirtschaft, in Forsten, im Vorrats- und Materialschutz sowie auf dem Hygienesektor vorkommen. Sie können vorzugsweise als Pflanzenschutzmittel eingesetzt werden. Sie sind gegen normal sensible und resistente Arten sowie gegen alle oder einzelne Entwicklungsstadien wirksam. Zu den oben erwähnten Schädlingen gehören:
Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophagus, Scutigera spp.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Acheta domesticus, Gryllotalpa spp., Locusta migratoria migratorioides, Melanoplus spp., Schistocerca gregaria.
Aus der Ordnung der Blattaria z.B. Blatta orientalis, Periplaneta americana, Leucophaea maderae, Blattella germanica.
Aus der Ordnung der Dermaptera z.B. Forficula auricularia.
Aus der Ordnung der Isoptera z.B. Reticulitermes spp.
Aus der Ordnung der Phthiraptera z.B. Pediculus humanus corporis, Haematopinus spp., Linognathus spp., Trichodectes spp., Damalinia spp.
Aus der Ordnung der Thysanoptera z.B. Hercinothrips femoralis, Thrips tabaci, Thrips palmi, Frankliniella accidentalis.
Aus der Ordnung der Heteroptera z.B. Eurygaster spp., Dysdercus intermedius, Piesma quadrata, Cimex lectularius, Rhodnius prolixus, Triatoma spp.
Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Aphis pomi, Eriosoma lanigerum, Hyalopterus arundinis, Phylloxera vastatrix, Pemphigus spp., Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp., Psylla spp.
Aus der Ordnung der Lepidoptera z.B. Pectinophora gossypiella, Bupalus piniarius, Cheimatobia brumata, Lithocolletis blancardella, Hyponomeuta padella, Plutella xylostella, Malacosoma neustria, Euproctis chrysorrhoea, Lymantria spp., Bucculatrix thurberiella, Phyllocnistis citrella, Agrotis spp., Euxoa spp., Feltia spp., Earias insulana, Heliothis spp., Mamestra brassicae, Panolis flammea, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana, Cnaphalocerus spp.
Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.
Aus der Ordnung der Hymenoptera z.B. Diprion spp., Hoplocampa spp., Lasius spp., Monomorium pharaonis, Vespa spp.
Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa, Hylemyia spp., Liriomyza spp..
Aus der Ordnung der Siphonaptera z.B. Xenopsylla cheopis, Ceratophyllus spp.
Aus der Klasse der Arachnida z.B. Scorpio maurus, Latrodectus mactans, Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp., Hemitarsonemus spp., Brevipalpus spp.

Zu den pflanzenparasitären Nematoden gehören z.B. Pratylenchus spp., Radopholus similis, Ditylenchus dipsaci, Tylenchulus semipenetrans, Heterodera spp., Globodera spp., Meloidogyne spp., Aphelenchoides spp., Longidorus spp., Xiphinema spp., Trichodorus spp., Bursaphelenchus spp.

Sie lassen sich mit besonders gutem Erfolg zur Bekämpfung von pflanzenschädigenden Insekten, wie beispielsweise gegen die Raupen der Kohlschabe (Plutella maculipennis) einsetzen.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Einweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90 %.

Die erfindungsgemäßen Wirkstoffe können als solche oder in ihren Formulierungen auch in Mischung mit bekannten Fungiziden, Bakteriziden, Akariziden, Nematiziden oder Insektiziden verwendet werden, um so z.B. das Wirkungsspektrum zu verbreitern oder Resistenzentwicklungen vorzubeugen. In vielen Fällen erhält man dabei synergistische Effekte, d.h. die Wirksamkeit der Mischung ist größer als die Wirksamkeit der Einzelkomponenten.

Besonders günstige Mischpartner sind z.B. die folgenden:

### Fungizide:

Aldimorph, Ampropylfos, Ampropylfos-Kalium, Andoprim, Anilazin, Azaconazol, Azoxystrobin,
Benalaxyl, Benodanil, Benomyl, Benzamacril, Benzamacryl-isobutyl, Bialaphos, Binapacryl, Biphenyl, Bitertanol, Blasticidin-S, Bromuconazol, Bupirimat, Buthiobat,
Calciumpolysulfid, Capsimycin, Captafol, Captan, Carbendazim, Carboxin, Carvon, Chinomethionat (Quinomethionat), Chlobenthiazon, Chlorfenazol, Chloroneb, Chloropicrin, Chlorothalonil, Chlozolinat, Clozylacon, Cufraneb, Cymoxanil, Cyproconazol, Cyprodinil, Cyprofuram,
Debacarb, Dichlorophen, Diclobutrazol, Diclofluanid, Diclomezin, Dicloran, Diethofencarb, Difenoconazol, Dimethirimol, Dimethomorph, Diniconazol, Diniconazol-M, Dinocap, Diphenylamin, Dipyrithione, Ditalimfos, Dithianon, Dodemorph, Dodine, Drazoxolon,
Ediphenphos, Epoxiconazol, Etaconazol, Ethirimol, Etridiazol,
Famoxadon, Fenapanil, Fenarimol, Fenbuconazol, Fenfuram, Fenitropan, Fenpiclonil, Fenpropidin, Fenpropimorph, Fentinacetat, Fentinhydroxyd, Ferbam, Ferimzon, Fluazinam, Flumetover, Fluoromid, Fluquinconazol, Flurprimidol, Flusilazol, Flusulfamid, Flutolanil, Flutriafol, Folpet, Fosetyl-Alminium, Fosetyl-Natrium, Fthalid, Fuberidazol, Furalaxyl, Furametpyr, Furcarbonil, Furconazol, Furconazol-cis, Furmecyclox,
Guazatin,
Hexachlorobenzol, Hexaconazol, Hymexazol,
Imazalil, Imibenconazol, Iminoctadin, Iminoctadinealbesilat, Iminoctadinetriacetat, Iodocarb, Ipconazol, Iprobenfos (IBP), Iprodione, Irumamycin, Isoprothiolan, Isovaledione,
Kasugamycin, Kresoxim-methyl, Kupfer-Zubereitungen, wie: Kupferhydroxid, Kupfemaphthenat, Kupferoxychlorid, Kupfersulfat, Kupferoxid, Oxin-Kupfer und Bordeaux-Mischung,
Mancopper, Mancozeb, Maneb, Meferimzone, Mepanipyrim, Mepronil, Metalaxyl, Metconazol, Methasulfocarb, Methfuroxam, Metiram, Metomeclam, Metsulfovax, Mildiomycin, Myclobutanil, Myclozolin,
Nickel-dimethyldithiocarbamat, Nitrothal-isopropyl, Nuarimol,
Ofurace, Oxadixyl, Oxamocarb, Oxolinicacid, Oxycarboxim, Oxyfenthiin,
Paclobutrazol, Pefurazoat, Penconazol, Pencycuron, Phosdiphen, Pimaricin, Piperalin, Polyoxin, Polyoxorim, Probenazol, Prochloraz, Procymidon, Propamocarb, Propanosine-Natrium, Propiconazol, Propineb, Pyrazophos, Pyrifenox, Pyrimethanil, Pyroquilon, Pyroxyfur,
Quinconazol, Quintozen (PCNB), Quinoxyfen,
Schwefel und Schwefel-Zubereitungen,
Tebuconazol, Tecloftalam, Tecnazen, Tetcyclacis, Tetraconazol, Thiabendazol, Thicyofen, Thifluzamide, Thiophanate-methyl, Thiram, Tioxymid, Tolclofos-methyl, Tolylfluanid, Triadimefon, Triadimenol, Triazbutil, Triazoxid, Trichlamid, Tricyclazol, Tridemorph, Triflumizol, Triforin, Triticonazol,
Uniconazol,
Validamycin A, Vinclozolin, Viniconazol,
Zarilamid, Zineb, Ziram sowie
Dagger G,
OK-8705,
OK-8801,
α-(1,1-Dimethylethyl)-β-(2-phenoxyethyl)-1H-1,2,4-triazol-1-ethanol,
α-(2,4-Dichlorphenyl)-β-fluor-b-propyl-1H-1,2,4-triazol-1-ethanol,
α-(2,4-Dichlorphenyl)-β-methoxy-a-methyl-1H-1,2,4-triazol-1-ethanol,
α-(5-Methyl-1,3-dioxan-5-yl)-β-[[4-(trifluormethyl)-phenyl]-methylen]-1H-1,2,4-triazol-1-ethanol,
(5RS,6RS)-6-Hydroxy-2,2,7,7-tetramethyl-5-(1H-1,2,4-triazol-1-yl)-3-octanon,
(E)-a-(Methoxyimino)-N-methyl-2-phenoxy-phenylacetamid,
{2-Methyl-1-[[[1-(4-methylphenyl)-ethyl]-amino]-carbonyl]-propyl}-carbaminsäure-1-isopropylester
1-(2,4-Dichlorphenyl)-2-(1H-1,2,4-triazol-1-yl)-ethanon-O-(phenylmethyl)-oxim,
1-(2-Methyl-1-naphthalenyl)-1H-pyrrol-2,5-dion,
1-(3,5-Dichlorphenyl)-3-(2-propenyl)-2,5-pyrrolidindion,
1-[(Diiodmethyl)-sulfonyl]-4-methyl-benzol,
1-[[2-(2,4-Dichlorphenyl)-1,3-dioxolan-2-yl]-methyl]-1H-imidazol,
1-[[2-(4-Chlorphenyl)-3-phenyloxiranyl]-methyl]-1H-1,2,4-triazol,
1-[1-[2-[(2,4-Dichlorphenyl)-methoxy]-phenyl]-ethenyl]-1H-imidazol,
1-Methyl-5-nonyl-2-(phenylmethyl)-3-pyrrolidinol,
2',6'-Dibrom-2-methyl-4'-trifluormethoxy-4'-trifluor-methyl-1,3-thiazol-5-carboxanilid,
2,2-Dichlor-N-[1-(4-chlorphenyl)-ethyl]-1-ethyl-3-methyl-cyclopropancarboxamid,
2,6-Dichlor-5-(methylthio)-4-pyrimidinyl-thiocyanat,
2,6-Dichlor-N-(4-trifluormethylbenzyl)-benzamid,
2,6-Dichlor-N-[[4-(trifluormethyl)-phenyl]-methyl]-benzamid,
2-(2,3,3-Triiod-2-propenyl)-2H-tetrazol,
2-[(1-Methylethyl)-sulfonyl]-5-(trichlormethyl)-1,3,4-thiadiazol,
2-[[6-Deoxy-4-O-(4-O-methyl-β-D-glycopyranosyl)-a-D-glucopyranosyl]-amino]-4-methoxy-1H-pyrrolo[2,3-d]pyrimidin-5-carbonitril,
2-Aminobutan,
2-Brom-2-(brommethyl)-pentandinitril,
2-Chlor-N-(2,3-dihydro-1,1,3-trimethyl-1H-inden-4-yl)-3-pyridincarboxamid,
2-Chlor-N-(2,6-dimethylphenyl)-N-(isothiocyanatomethyl)-acetamid,
2-Phenylphenol(OPP),
3,4-Dichlor-1-[4-(difluormethoxy)-phenyl]-1H-pyrrol-2,5-dion,
3,5-Dichlor-N-[cyan[(1-methyl-2-propynyl)-oxy]-methyl]-benzamid,
3-(1,1-Dimethylpropyl-1-oxo-1H-inden-2-carbonitril,
3-[2-(4-Chlorphenyl)-5-ethoxy-3-isoxazolidinyl]-pyridin,
4-Chlor-2-cyan-N,N-dimethyl-5-(4-methylphenyl)-1H-imidazol-1-sulfonamid,
4-Methyl-tetrazolo[ 1,5-a]quinazolin-5(4H)-on,
8-(1,1-Dimethylethyl)-N-ethyl-N-propyl-1,4-dioxaspiro[4.5]decan-2-methanamin,
8-Hydroxychinolinsulfat,
9H-Xanthen-9-carbonsäure-2-[(phenylamino)-carbonyl]-hydrazid,
bis-(1-Methylethyl)-3-methyl-4-[(3-methylbenzoyl)-oxy]-2,5-thiophendicarboxylat,
cis-1-(4-Chlorphenyl)-2-(1H-1,2,4-triazol-1-yl)-cycloheptanol,
cis-4-[3-[4-(1,1-Dimethylpropyl)-phenyl-2-methylpropyl]-2,6-dimethyl-morpholinhydrochlorid,
Ethyl-[(4-chlorphenyl)-azo]-cyanoacetat,
Kaliumhydrogencarbonat,
Methantetrathiol-Natriumsalz,
Methyl-1-(2,3-dihydro-2,2-dimethyl-1H-inden-1-yl)-1H-imidazol-5-carboxylat,
Methyl-N-(2,6-dimethylphenyl)-N-(5-isoxazolylcarbonyl)-DL-alaninat,
Methyl-N-(chloracetyl)-N-(2,6-dimethylphenyl)-DL-alaninat,
N-(2,3-Dichlor-4-hydroxyphenyl)-1-methyl-cyclohexancarboxamid.
N-(2,6-Dimethylphenyl)-2-methoxy-N-(tetrahydro-2-oxo-3-furanyl)-acetamid,
N-(2,6-Dimethylphenyl)-2-methoxy-N-(tetrahydro-2-oxo-3-thienyl)-acetamid,
N-(2-Chlor-4-nitrophenyl)-4-methyl-3-nitro-benzolsulfonamid,
N-(4-Cyclohexylphenyl)-1,4,5,6-tetrahydro-2-pyrimidinamin,
N-(4-Hexylphenyl)-1,4,5,6-tetrahydro-2-pyrimidinamin,
N-(5-Chlor-2-methylphenyl)-2-methoxy-N-(2-oxo-3-oxazolidinyl)-acetamid,
N-(6-Methoxy)-3-pyridinyl)-cyclopropancarboxamid,
N-[2,2,2-Trichlor-1-[(chloracetyl)-amino]-ethyl]-benzamid,
N-[3-Chlor-4,5-bis-(2-propinyloxy)-phenyl]-N'-methoxy-methanimidamid,
N-Formyl-N-hydroxy-DL-alanin -Natriumsalz,
O,O-Diethyl-[2-(dipropylamino)-2-oxoethyl]-ethy]phosphoramidothioat,
O-Methyl-S-phenyl-phenylpropylphosphoramidothioat,
S-Methyl-1,2,3-benzothiadiazol-7-carbothioat,
spiro[2H]-1-Benzopyran-2,1'(3'H)-isobenzofuran]-3'-on,

### Bakterizide:

Bronopol, Dichlorophen, Nitrapyrin, Nickel-Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide:

Abamectin, Acephate, Acetamiprid, Acrinathrin, Alanycarb, Aldicarb, Aldoxycarb, Alpha-cypermethrin, Alphamethrin, Amitraz, Avermectin, AZ 60541, Azadirachtin, Azamethiphos, Azinphos A, Azinphos M, Azocyclotin,
Bacillus popilliae, Bacillus sphaericus, Bacillus subtilis, Bacillus thuringiensis, Baculoviren, Beauveria bassiana, Beauveria tenella, Bendiocarb, Benfuracarb, Bensultap, Benzoximate, Betacyfluthrin, Bifenazate, Bifenthrin, Bioethanomethrin, Biopermethrin, BPMC, Bromophos A, Bufencarb, Buprofezin, Butathiofos, Butocarboxim, Butylpyridaben,
Cadusafos, Carbaryl, Carbofuran, Carbophenothion, Carbosulfan, Cartap, Chloethocarb, Chlorethoxyfos, Chlorfenapyr, Chlorfenvinphos, Chlorfluazuron, Chlormephos, Chlorpyrifos, Chlorpyrifos M, Chlovaporthrin, Cis-Resmethrin, Cispermethrin, Clocythrin, Cloethocarb, Clofentezine, Cyanophos, Cycloprene, Cycloprothrin, Cyfluthrin, Cyhalothrin, Cyhexatin, Cypermethrin, Cyromazine,
Deltamethrin, Demeton M, Demeton S, Demeton-S-methyl, Diafenthiuron, Diazinon, Dichlorvos, Diflubenzuron, Dimethoat, Dimethylvinphos, Diofenolan, Disulfoton, Docusat-sodium, Dofenapyn,
Eflusilanate, Emamectin, Empenthrin, Endosulfan, Entomopfthora spp., Esfenvalerate, Ethiofencarb, Ethion, Ethoprophos, Etofenprox, Etoxazole, Etrimfos,
Fenamiphos, Fenazaquin, Fenbutatin oxide, Fenitrothion, Fenothiocarb, Fenoxacrim, Fenoxycarb, Fenpropathrin, Fenpyrad, Fenpyrithrin, Fenpyroximate, Fenvalerate, Fipronil, Fluazinam, Fluazuron, Flubrocythrinate, Flucycloxuron, Flucythrinate, Flufenoxuron, Flutenzine, Fluvalinate, Fonophos, Fosmethilan, Fosthiazate, Fubfenprox, Furathiocarb,
Granuloseviren
Halofenozide, HCH, Heptenophos, Hexaflumuron, Hexythiazox, Hydroprene,
Imidacloprid, Isazofos, Isofenphos, Isoxathion, Ivermectin,
Kernpolyederviren
Lambda-cyhalothrin, Lufenuron
Malathion, Mecarbam, Metaldehyd, Methamidophos, Metharhizium anisopliae, Metharhizium flavoviride, Methidathion, Methiocarb, Methomyl, Methoxyfenozide, Metolcarb, Metoxadiazone, Mevinphos, Milbemectin, Monocrotophos,
Naled, Nitenpyram, Nithiazine, Novaluron
   Omethoat, Oxamyl, Oxydemethon M
   Paecilomyces fumosoroseus, Parathion A, Parathion M, Permethrin, Phenthoat, Phorat, Phosalone, Phosmet, Phosphamidon, Phoxim, Pirimicarb, Pirimiphos A, Pirimiphos M, Profenofos, Promecarb, Propoxur, Prothiofos, Prothoat, Pymetrozine, Pyraclofos, Pyresmethrin, Pyrethrum, Pyridaben, Pyridathion, Pyrimidifen, Pyriproxyfen,
   Quinalphos,
Ribavirin
Salithion, Sebufos, Silafluofen, Spinosad, Sulfotep, Sulprofos,
Tau-fluvalinate, Tebufenozide, Tebufenpyrad, Tebupirimiphos, Teflubenzuron, Tefluthrin, Temephos, Temivinphos, Terbufos, Tetrachlorvinphos, Theta-cypermethrin, Thiamethoxam, Thiapronil, Thiatriphos, Thiocyclam hydrogen oxalate, Thiodicarb, Thiofanox, Thuringiensin, Tralocythrin, Tralomethrin, Triarathene, Triazamate, Triazophos, Triazuron, Trichlophenidine, Trichlorfon, Triflumuron, Trimethacarb,
Vamidothion, Vaniliprole, Verticillium lecanii
Zeta-cypermethrin, Zolaprofos
(1R-cis)-[5-(Phenylmethyl)-3-furanyl]-methyl-3-[(dihydro-2-oxo-3(2H)-furanyliden)-methyl]-2,2-dimethylcyclopropancarboxylat
(3-Phenoxyphenyl)-methyl-2,2,3,3-tetramethylcyclopropanecarboxylat
1-[(2-Chlor-5-thiazolyl)methyl]tetrahydro-3,5-dimethyl-N-nitro-1,3,5-triazin-2(1H)-imin
2-(2-Chlor-6-fluorphenyl)-4-[4-(1,1-dimethylethyl)phenyl]-4,5-dihydro-oxazol
2-(Acetlyoxy)-3-dodecyl-1,4-naphthalindion
2-Chlor-N-[[[4-(1-phenylethoxy)-phenyl]-amino]-carbonyl]-benzamid
2-Chlor-N-[[[4-(2,2-dichlor-1,1-difluorethoxy)-phenyl]-amino]-carbonyl]-benzamid
3-Methylphenyl-propylcarbamat
4-[4-(4-Ethoxyphenyl)-4-methylpentyl]-1-fluor-2-phenoxy-benzol
4-Chlor-2-(1,1-dimethylethyl)-5-[[2-(2,6-dimethyl-4-phenoxyphenoxy)ethyl]thio]-3(2H)-pyridazinon
4-Chlor-2-(2-chlor-2-methylpropyl)-5-[(6-iod-3-pyridinyl)methoxy]-3(2H)-pyridazinon
4-Chlor-5-[(6-chlor-3-pyridinyl)methoxy]-2-(3,4-dichlorphenyl)-3(2H)-pyridazinon
Bacillus thuringiensis strain EG-2348
Benzoesäure [2-benzoyl-1-(1,1-dimethylethyl)-hydrazid
Butansäure 2,2-dimethyl-3-(2,4-dichlorphenyl)-2-oxo-1-oxaspiro[4.5]dec-3-en-4-ylester
[3-[(6-Chlor-3-pyridinyl)methyl]-2-thiazolidinyliden]-cyanamid
Dihydro-2-(nitromethylen)-2H-1,3-thiazine-3(4H)-carboxaldehyd
Ethyl-[2-[[1,6-dihydro-6-oxo-1-(phenylmethyl)-4-pyridazinyl]oxy]ethyl]-carbamat
N-(3,4,4-Trifluor-1-oxo-3-butenyl)-glycin
N-(4-Chlorphenyl)-3-[4-(difluormethoxy)phenyl]-4,5-dihydro-4-phenyl-1H-pyrazol-1-carboxamid
N-[(2-Chlor-5-thiazolyl)methyl]-N'-methyl-N"-nitro-guanidin
N-Methyl-N'-(1-methyl-2-propenyl)-1,2-hydrazindicarbothioamid
N-Methyl-N'-2-propenyl-1,2-hydrazindicarbothioamid
O,O-Diethyl-[2-(dipropylamino)-2-oxoethyl]-ethylphosphoramidothioat

Auch eine Mischung mit anderen bekannten Wirkstoffen, wie Herbiziden oder mit Düngemitteln und Wachstumsregulatoren ist möglich.

Die erfindungsgemäßen Wirkstoffe können ferner beim Einsatz als Insektizide in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne dass der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnet sich der Wirkstoff durch eine hervorragende Residualwirkung auf Holz und Ton sowie durch eine gute Alkalistabilität auf gekälkten Unterlagen aus.

Die Herstellung und die Verwendung der erfindungsgemäßen Wirkstoffe geht aus den nachfolgenden Beispielen hervor.

Die nachfolgenden Beispiele dienen zur Erläuterung der Erfindung. Die Erfindung ist jedoch nicht auf die Beispiele limitiert.

### Herstellungsbeispiele:

### Beispiel 1

### 2-{2-[6-(3-Fluorphenoxy)-5-fluor-pyrimidin-4-yloxy]-phenyl}-2-methoximinoessigsäuremethylester

### Verfahren a)

Zu einer Mischung aus 4,6 g (0,0221 mol) 2-(2-Hydroxyphenyl)-2-methoxyiminoessigsäuremethylester und 5 g (0,0221 mol) 4-(3-Fluorphenoxy)-5,6-difluorpyrimidin in 50 ml Acetonitril gibt man unter Kühlen 4 g (0,0287 mol) Kaliumcarbonat zu und rührt 12 Stunden bei 25°C. Man gießt das Reaktionsgemisch auf 400 ml Wasser, extrahiert dreimal mit jeweils 150 ml Essigsäureethylester, trocknet die organische Phase über Natriumsulfat und destilliert das Lösungsmittel im Vakuum ab. Der Rückstand wird mit Diisopropylether verrührt, der dabei entstehende Feststoff wird abfiltriert und getrocknet. Man erhält 7,3 g (79,5 % der Theorie) 2-{2-[6-(3-Fluorphenoxy)-5-fluor-pyrimidin-4-yloxy]-phenyl}-2-methoxyiminoessigsäuremethylester.
HPLC: logP = 3,46

Analog Beispiel 1, sowie entsprechend den Angaben in der allgemeinen Verfahrensbeschreibung, werden die in der nachstehenden Tabelle 1 genannten Verbindungen der Formel (I-a) erhalten.

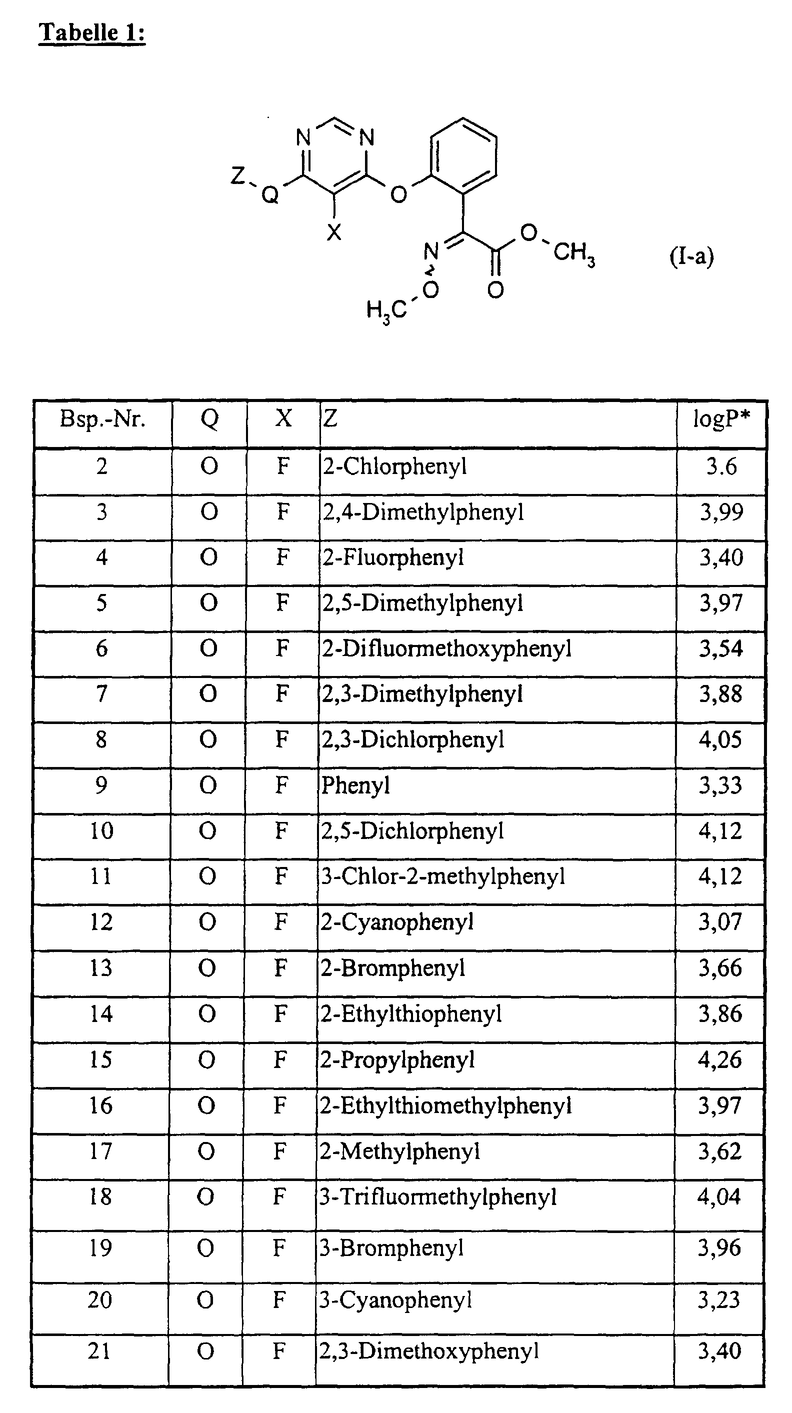

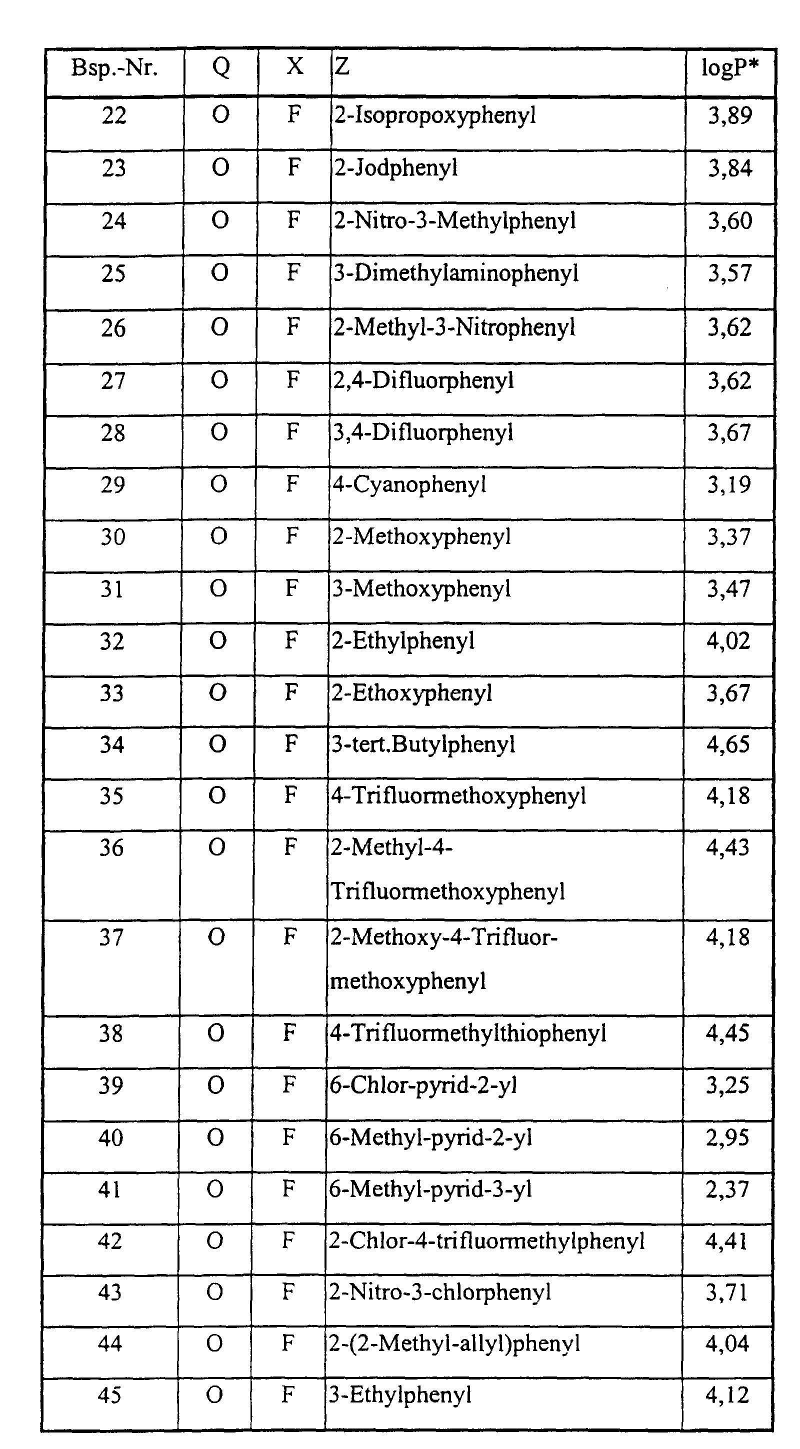

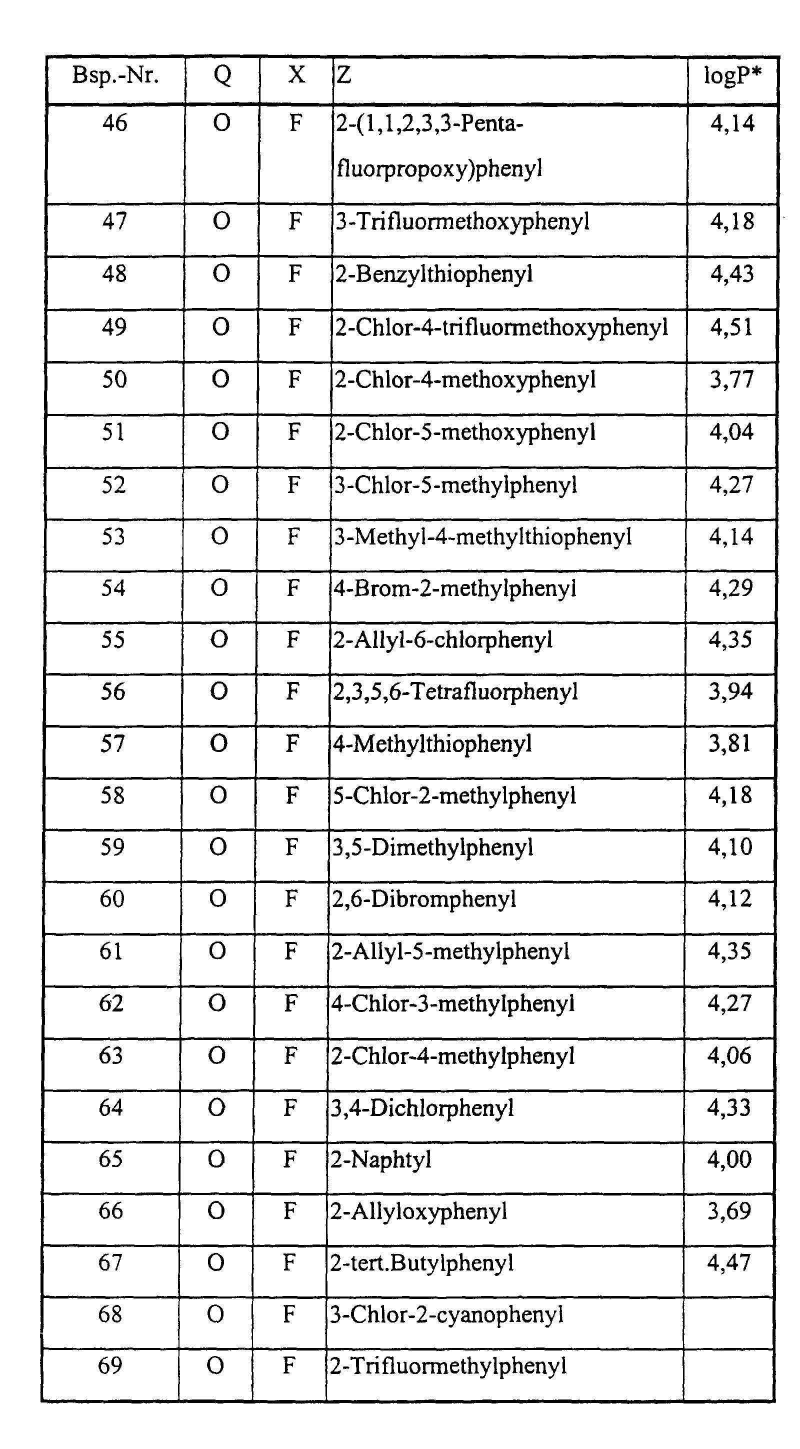

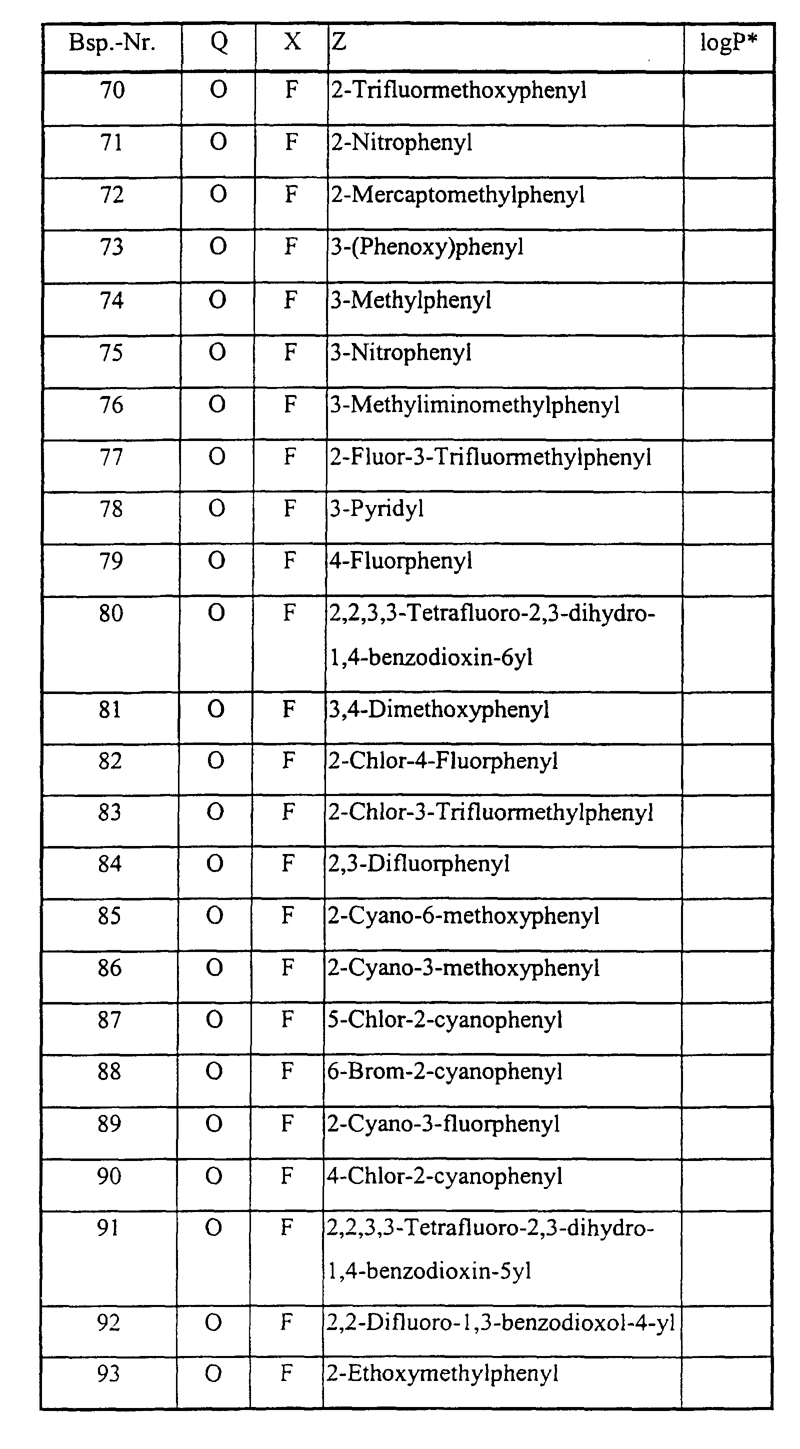

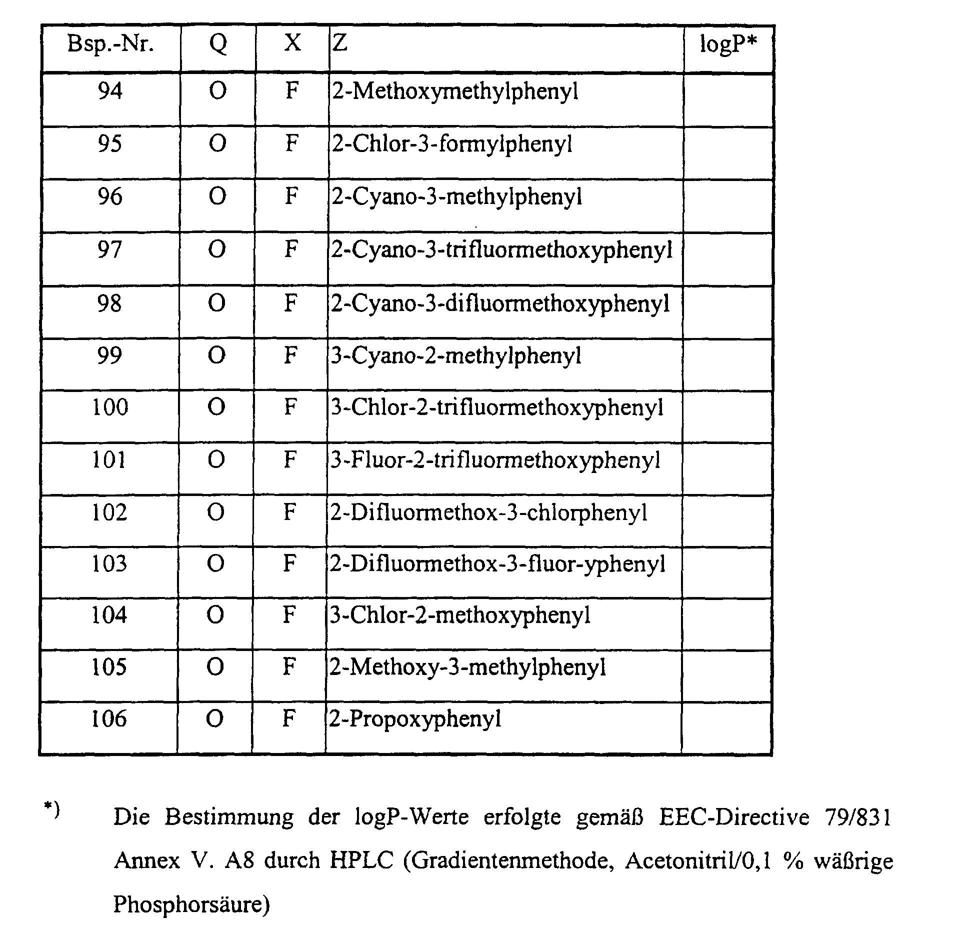

### Herstellung eines Ausgangsstoffes nach Formel (III):

### Beispiel (III-1)

### 5,6-Difluor-(3-fluorphenoxy)-pyrimidin

11,38 g (0,085 mol) Trifluorpyrimidin werden in 240 ml Acetonitril gelöst, mit 15,26 g (0,11 mol) Kaliumcarbonat versetzt und auf 10°C gekühlt. Hierzu tropft man unter Argon eine Lösung von 9,52 g (0,085 mol) 3-Fluorphenol in 80 ml Acetonitril. Hierauf rührt man die Mischung noch 18 Stunden ohne weitere Kühlung unter Argon. Die Mischung wird auf 1 Liter Wasser gegossen, dreimal mit jeweils 150 ml Essigsäureethylester extrahiert, die organische Phase über Natriumsulfat getrocknet und im Vakuum abdestilliert. Der Rückstand wird noch einer Kugelrohrdestillation unterzogen. Man erhält 15,2 g (79,1 % der Theorie) 5,6-Difluor-(3-fluorphenoxy)-pyrimidin vom Siedepunkt 95 °C bei 0,5 mbar.

### Anwendungsbeispiele

### Beispiel A

### Leptosphaeria nodorum-Test (Weizen) / protektiv

| | |
|---|---|
| Lösungsmittel | 25 Gewichtsteile N,N-Dimethylacetamid |
| Emulgator | 0,6 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer Sporensuspension von Leptosphaeria nodorum besprüht. Die Pflanzen verbleiben 48 Stunden bei 20°C und 100 % relativer Luftfeuchtigkeit in einer Inkubationskabine.

Die Pflanzen werden in einem Gewächshaus bei einer Temperatur von ca. 15°C und einer relativen Luftfeuchtigkeit von 80 % aufgestellt.

10 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

Bei diesem Test zeigen die in den Beispielen (6), (8) und (17) aufgeführten erfindungsgemäßen Stoffe bei einer Aufwandmenge von 250 g/ha einen Wirkungsgrad von 95 % oder mehr.

### Beispiel B

### Phytophthora-Test (Tomate) / protektiv

| | |
|---|---|
| Lösungsmittel | 24,5 Gewichtsteile Aceton |
| | 24,5 Gewichtsteile Dimethylacetamid |
| Emulgator | 1,0 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wäßrigen Sporensuspension von Phytophthora infestans inokuliert. Die Pflanzen werden dann in einer Inkubationskabine bei ca. 20°C und 100 % relativer Luftfeuchtigkeit aufgestellt.

3 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

Bei diesem Test zeigen die in den Beispielen (3), (4), (7), (11) und (17) aufgeführten erfindungsgemäßen Stoffe bei einer Aufwandmenge von 100 g/ha einen Wirkungsgrad von 90 % oder mehr.

### Beispiel C

### Plasmopara-Test (Rebe) / protektiv

| | |
|---|---|
| Lösungsmittel | 24,5 Gewichtsteile Aceton |
| | 24,5 Gewichtsteile Dimethylacetamid |
| Emulgator | 1,0 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wäßrigen Sporensuspension von Plasmopara viticola inokuliert und verbleiben dann 1 Tag in einer Inkubationskabine bei ca. 20°C und 100 % relativer Luftfeuchtigkeit. Anschließend werden die Pflanzen 5 Tage im Gewächshaus bei ca. 21°C und ca. 90 % relativer Luftfeuchtigkeit aufgestellt. Die Pflanzen werden dann angefeuchtet und 1 Tag in eine Inkubationskabine gestellt.

6 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

Bei diesem Test zeigen die in den Beispielen (4), (7), (11) und (17) aufgeführten erfindungsgemäßen Stoffe bei einer Aufwandmenge von 100g/ha einen Wirkungsgrad von 95 % oder mehr.

### Beispiel D

### Venturia-Test (Apfel) / protektiv

| | |
|---|---|
| Lösungsmittel | 24,5 Gewichtsteile Aceton |
| | 24,5 Gewichtsteile Dimethylacetamid |
| Emulgator | 1,0 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wäßrigen Konidiensuspension des Apfelschorferregers ***Venturia inaequalis*** inokuliert und verbleiben dann 1 Tag bei ca. 20°C und 100 % relativer Luftfeuchtigkeit in einer Inkubationskabine.

Die Pflanzen werden dann im Gewächshaus bei ca. 21°C und einer relativen Luftfeuchtigkeit von ca. 90 % aufgestellt.

12 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

Bei diesem Test zeigen die in den Beispielen (3), (4), (7), (11) und (17) aufgeführten erfindungsgemäßen Stoffe bei einer Aufwandmenge von 10g/ha einen Wirkungsgrad von 90 % oder mehr.

### Beispiel E

### Pyricularia-Test (Reis) / protektiv

| | |
|---|---|
| Lösungsmittel | 25 Gewichtsteile N,N-Dimethylacetamid |
| Emulgator | 0,6 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Reispflanzen mit der Wirkstoffzubereitung in der angegebenen Aufwandmenge. Nach dem Antrocknen des Spritzbelages werden die Pflanzen mit einer wäßrigen Sporensuspension von *Pyricularia oryzae* inokuliert und verbleiben dann 24 h bei 100 % rel. Luftfeuchte und 26°C. Anschließend werden die Pflanzen in einem Gewächshaus bei 80 % rel. Luftfeuchtigkeit und einer Temperatur von 26°C aufgestellt.

7 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

Bei diesem Test zeigen die in den Beispielen (4), (6), (7), (8), (11) und (17) aufgeführten erfindungsgemäßen Stoffe bei einer Aufwandmenge von 125g/ha einen Wirkungsgrad von 90 % oder mehr.

### Beispiel F

### Sphaerotheca-Test (Gurke) / protektiv

| | |
|---|---|
| Lösungsmittel | 50 Gewichtsteile N,N-Dimethylformamid |
| Emulgator | 1,17 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und verdünnt das Konzentrat mit Wasser und der angegebenen Menge Emulgator und auf die gewünschte Konzentration.

Zur Prüfung auf protektive Wirksamkeit bespritzt man junge Gurkenpflanzen mit der Wirkstoffzubereitung in der angegebenen Aufwandmenge. 1 Tag nach der Behandlung werden die Pflanzen mit einer wäßrigen Sporensuspension von *Sphaerotheca fuliginea* inokuliert. Anschließend werden die Pflanzen in einem Gewächshaus bei 70 % relativer Luftfeuchtigkeit und einer Temperatur von 23°C aufgestellt.

7 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

Bei diesem Test zeigen die in den Beispielen (4), (5), (7), (8), (9), (11) und (17) aufgeführten erfindungsgemäßen Stoffe bei einer Aufwandmenge von 750 g/ha einen Wirkungsgrad von 90 % oder mehr.

### Beispiel G

### Plutella-Test/Kunstfutter

| | |
|---|---|
| Lösungsmittel | 100 Gewichtsteile Aceton |
| Emulgator | 1900 Gewichtsteile Methanol |

Zur Herstellung einer zweckmäßigen Wirkstoffzubereitung vermischt man 1 Gewichtsteil Wirkstoff mit der angegebenen Menge Lösungsmittel und verdünnt das Konzentrat mit Methanol auf die gewünschte Konzentration.

Auf einer genormten Menge Kunstfutter wird eine angegebene Menge Wirkstoffzubereitung der gewünschten Konzentration pipettiert. Nachdem das Methanol verdunstet ist, wird je Kavität ein mit ca. 100 Plutella-Eiern belegte Filmdosendeckel aufgesetzt. Die frisch geschlüpften Larven wandern auf das behandelte Kunstfutter.

Nach der gewünschten Zeit wird die Abtötung in % bestimmt. Dabei bedeutet 100 %, dass alle Tiere abgetötet wurden; 0 % bedeutet, dass keine Tiere abgetötet wurden.

Bei diesem Test zeigt z.B. die folgende Verbindung der Herstellungsbeispiele gute Wirksamkeit: (10)

## Patentansprüche

1. Halogenpyrimidine der allgemeinen Formel (I), 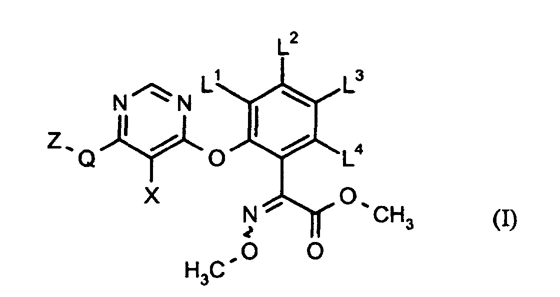 in welcher
Z für jeweils gegebenenfalls einfach bis zweifach durch Halogen, Alkyl, oder Hydroxy substituiertes Cycloalkyl mit 3 bis 7 Kohlenstoffatomen;
für gegebenenfalls durch Alkyl mit 1 bis 4 Kohlenstoffatomen oder Halogen substituiertes Heterocyclyl mit 3 bis 7 Ringgliedern;
oder für jeweils gegebenenfalls einfach bis vierfach, gleich oder verschieden substituiertes Phenyl oder Naphthyl steht, wobei die möglichen Substituenten vorzugsweise aus der nachstehenden Aufzählung ausgewählt sind:
Halogen, Cyano, Nitro, Amino, Hydroxy, Formyl, Carboxy, Carbamoyl, Thiocarbamoyl;
jeweils geradkettiges oder verzweigtes Alkyl, Hydroxyalkyl, Oxoalkyl, Alkoxy, Alkoxyalkyl, Alkylthioalkyl, Dialkoxyalkyl, Alkylthio, Mercaptoalkyl, Alkylsulfinyl oder Alkylsulfonyl mit jeweils 1 bis 8 Kohlenstoffatomen;
jeweils geradkettiges oder verzweigtes Alkenyl oder Alkenyloxy mit jeweils 2 bis 6 Kohlenstoffatomen;
jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen;
jeweils geradkettiges oder verzweigtes Halogenalkenyl oder Halogenalkenyloxy mit jeweils 2 bis 6 Kohlenstoffatomen und 1 bis 11 gleichen oder verschiedenen Halogenatomen;
jeweils geradkettiges oder verzweigtes Alkylamino, Dialkylamino,
Alkylcarbonyl, Alkylcarbonyloxy, Alkoxycarbonyl, Alkylaminocarbonyl, Dialkylaminocarbonyl, Arylalkylaminocarbonyl, Dialkylaminocarbonyloxy, Alkenylcarbonyl oder Alkinylcarbonyl, mit 1 bis 6 Kohlenstoffatomen in den jeweiligen Kohlenwasserstoffketten;
Cycloalkyl oder Cycloalkyloxy mit jeweils 3 bis 6 Kohlenstoffatomen;
jeweils gegebenenfalls einfach bis vierfach, gleich oder verschieden durch Fluor, Chlor, Oxo, Methyl, Trifluormethyl oder Ethyl substituiertes, jeweils zweifach verknüpftes Alkylen mit 3 oder 4 Kohlenstoffatomen, Oxyalkylen mit 2 oder 3 Kohlenstoffatomen oder Dioxyalkylen mit 1 oder 2 Kohlenstoffatomen; oder eine Gruppierung 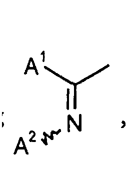 worin
A¹ für Wasserstoff, Hydroxy oder Alkyl mit 1 bis 4 Kohlenstoffatomen oder Cycloalkyl mit 1 bis 6 Kohlenstoffatomen steht und
A2 für Hydroxy, Amino, Methylamino, Phenyl, Benzyl oder für jeweils gegebenenfalls durch Cyano, Hydroxy, Alkoxy, Alkylthio, Alkylamino, Dialkylamino oder Phenyl substituiertes Alkyl oder Alkoxy mit 1 bis 4 Kohlenstoffatomen, oder für Alkenyloxy oder Alkinyloxy mit jeweils 2 bis 4 Kohlenstoffatomen steht,
sowie jeweils gegebenenfalls im Ringteil einfach bis dreifach durch Halogen, und/oder geradkettiges oder verzweigtes Alkyl oder Alkoxy mit 1 bis 4 Kohlenstoffatomen substituiertes Phenyl, Phenoxy, Phenylthio, Benzoyl, Benzoylethenyl, Cinnamoyl, Heterocyclyl oder Phenylalkyl, Phenylalkyloxy, Phenylalkylthio, oder Heterocyclylalkyl, mit jeweils 1 bis 3 Kohlenstoffatomen in den jeweiligen Alkylteilen,
Q für Sauerstoff oder Schwefel steht,
X für Fluor, Chlor oder Brom steht und
L¹, L², L³ und L⁴ gleich oder verschieden sind und unabhängig voneinander jeweils für Wasserstoff, Halogen, Cyano, Nitro, jeweils gegebenenfalls durch 1 bis 5 Halogenatome substituiertes Alkyl, Alkoxy, Alkylthio, Alkylsulfinyl oder Alkylsulfonyl mit jeweils 1 bis 6 Kohlenstoffatomen stehen.

2. Verbindungen der Formel (I), gemäß Anspruch 1, **dadurch gekennzeichnet, dass**
Z für jeweils gegebenenfalls einfach bis zweifach durch Fluor, Chlor, Methyl, Ethyl oder Hydroxy substituiertes Cyclopentyl oder Cyclohexyl;
für gegebenenfalls durch Methyl, Ethyl oder Chlor substituiertes Thienyl, Pyridyl oder Furyl;
oder für jeweils gegebenenfalls einfach bis vierfach, gleich oder verschieden substituiertes Phenyl oder Naphthyl steht, wobei die möglichen Substituenten vorzugsweise aus der nachstehenden Aufzählung ausgewählt sind:
Fluor, Chlor, Brom, Iod, Cyano, Nitro, Amino, Hydroxy, Formyl, Carboxy, Carbamoyl, Thiocarbamoyl,
Methyl, Ethyl, n- oder i-Propyl, n-, i-, s- oder t-Butyl, 1-, 2-, 3-, neo-Pentyl, 1-, 2-, 3-, 4-(2-Methylbutyl), 1-, 2-, 3-Hexyl, 1-, 2-, 3-, 4-, 5-(2-Methylpentyl), 1-, 2-, 3-(3-Methylpentyl), 2-Ethylbutyl, 1-, 3-, 4-(2,2-Dimetylbutyl), 1-, 2-(2,3-Dimethylbutyl), Hydroxymethyl, Hydroxyethyl, 3-Oxobutyl, Methoxymethyl, Dimethoxymethyl,
Methoxy, Ethoxy, n- oder i-Propoxy, Methoxymethyl, Ethoxymethyl,
Mercaptomethyl, Methylthio, Ethylthio, n- oder i-Propylthio, Methylsulfinyl, Ethylsulfinyl, Methylsulfonyl oder Ethylsulfonyl, Methylthiomethyl, Ethylthiomethyl,
Vinyl, Allyl, 2-Methylallyl, Propen-1-yl, Crotonyl, Propargyl, Vinyloxy, Allyloxy, 2-Methylallyloxy, Propen-1-yloxy, Crotonyloxy, Propargyloxy;
Trifluormethyl, Trifluorethyl,
Difluormethoxy, Trifluormethoxy, Difluorchlormethoxy, Trifluorethoxy, Difluormethylthio, Trifluormethylthio, Difluorchlormethylthio, Trifluormethylsulfinyl oder Trifluormethylsulfonyl,
Methylamino, Ethylamino, n- oder i-Propylamino, Dimethylamino, Diethylamino,
Acetyl, Propionyl, Methoxycarbonyl, Ethoxycarbonyl, Methylaminocarbonyl, Ethylaminocarbonyl, Dimethylaminocarbonyl, Diethylaminocarbonyl, Dimethylaminocarbonyloxy, Diethylaminocarbonyloxy, Benzylaminocarbonyl, Acryloyl, Propioloyl,
Cyclopentyl; Cyclohexyl,
jeweils gegebenenfalls einfach bis vierfach, gleich oder verschieden durch Fluor, Chlor, Oxo, Methyl oder Trifluormethyl substituiertes, jeweils zweifach verknüpftes Propandiyl, Ethylenoxy, Methylendioxy, Ethylendioxy
oder eine Gruppierung 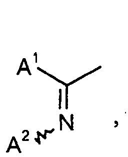 wobei
A¹ für Wasserstoff, Methyl oder Hydroxy steht und
A² für Hydroxy, Methoxy, Ethoxy, Amino, Methylamino, Phenyl, Benzyl oder Hydroxyethyl steht, sowie
jeweils gegebenenfalls im Ringteil einfach bis vierfach durch Halogen, und/oder geradkettiges oder verzweigtes Alkyl oder Alkoxy mit 1 bis 4 Kohlenstoffatomen substituiertes Phenyl, Phenoxy, Phenyllthio, Benzoyl, Benzoylethenyl, Cinnamoyl, Benzyl, Phenylethyl, Phenylpropyl, Benzyloxy, Benzylthio, 5,6-Dihydro-1,4,2-dioxazin-3-ylmethyl, Triazolylmethyl, Benzoxazol-2-ylmethyl, 1,3-Dioxan-2-yl, Benzimidazol-2-yl, Dioxol-2-yl, Oxadiazolyl,
Q für Sauerstoff steht,
X für Fluor steht und
L¹, L², L³ und L⁴ gleich oder verschieden sind und unabhängig voneinander jeweils für Wasserstoff, Methyl, Ethyl, n- oder i-Propyl, n-, i-, s- oder t-Butyl stehen.

3. Verbindungen der Formel (I), gemäß Anspruch 1, in denen Q für Sauerstoff steht.

4. Verbindungen der allgemeinen Formel (IV), 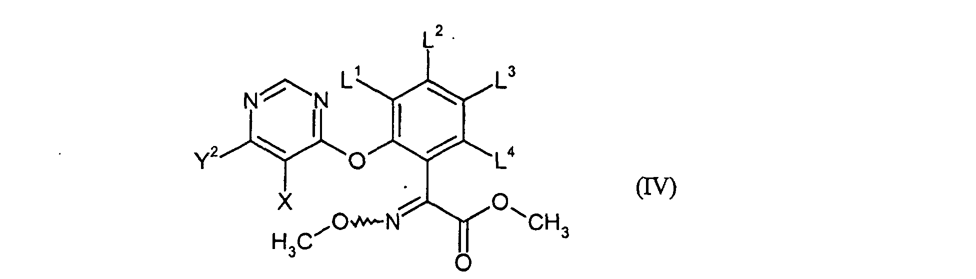 in welcher
X, L¹, L², L³ und L⁴ die in Anspruch 1 angegebenen Bedeutungen haben und
Y² für Halogen steht.

5. Mittel enthaltend Streckmittel und/oder Trägerstoffe sowie gegebenenfalls oberflächenaktive Stoffe, **gekennzeichnet durch** einen Gehalt an mindestens einer Verbindung wie in den Ansprüchen 1 bis 3 definiert.

6. Verfahren zur Bekämpfung von Schädlingen, **dadurch gekennzeichnet, dass** man Verbindungen wie in den Ansprüchen 1 bis 3 bzw. Mittel wie in Anspruch 5 definiert auf Schädlinge und/oder ihren Lebensraum einwirken lässt.

7. Verfahren zur Herstellung von Schädlingsbekämpfungsmitteln, **dadurch gekennzeichnet, dass** man Verbindungen der Formel (I) nach den Ansprüchen 1 bis 3 mit Streckmitteln und/oder oberflächenaktiven Mitteln vermischt.

8. Verwendung von Verbindungen wie in den Ansprüchen 1 bis 3 bzw. von Mitteln wie in Anspruch 5 definiert zur Bekämpfung von Schädlingen.

9. Verwendung von Verbindungen der Formel (IV) als Zwischenprodukte.

10. Verfahren zur Herstellung von Verbindungen der allgemeinen Formel (I), gemäß Anspruch 1, **dadurch gekennzeichnet, dass** man
a) 2-(2-Hydroxy-phenyl)-2-methoxyiminoessigester der Formel (II), 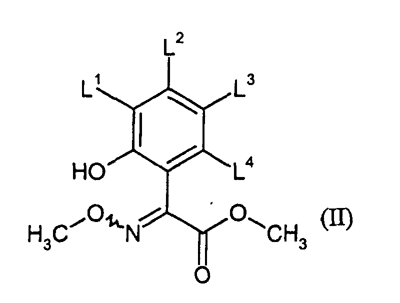 in welcher
L¹, L², L³ und L⁴ die in Anspruch 1 angegebenen Bedeutungen haben,
mit einem substituierten Halogenpyrimidin der allgemeinen Formel (III), 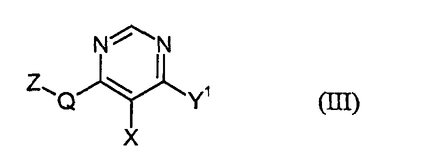 in welcher
Z, Q und X die in Anspruch 1 angegebenen Bedeutungen haben und
Y¹ für Halogen steht,
gegebenenfalls in Gegenwart eines Verdünnungsmittels, gegebenenfalls in Gegenwart eines Säureakzeptors und gegebenenfalls in Gegenwart eines Katalysators, umsetzt, oder
b) Phenoxypyrimidine der allgemeinen Formel (IV) 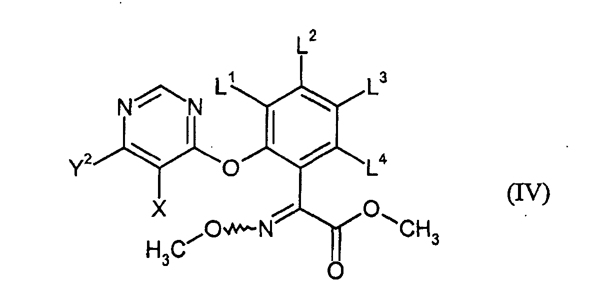
in welcher
X, L¹, L², L³ und L⁴ die in Anspruch 1 angegebenen Bedeutungen haben und
Y² für Halogen steht,
mit einer Ringverbindung der allgemeinen Formel (V),
Z-Q-H (V)
in welcher
Z und Q die oben angegebenen Bedeutungen haben,
gegebenenfalls in Gegenwart eines Verdünnungsmittels, gegebenenfalls in Gegenwart eines Säureakzeptors und gegebenenfalls in Gegenwart eines Katalysators, umsetzt.

11. Verfahren zur Herstellung von Verbindungen der Formel (IV) gemäß Anspruch 4, **dadurch gekennzeichnet, dass** man 2-(2-Hydroxy-phenyl)-2-methoxyimino-essigester der Formel (II), 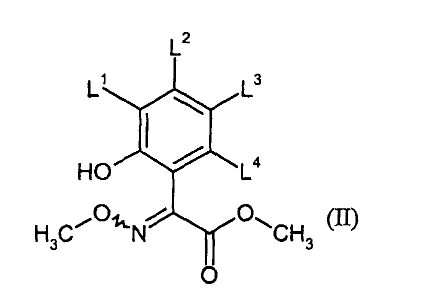 in welcher
L¹, L² , L³ und L⁴ die in Anspruch 1 angegebenen Bedeutungen haben,
mit einem Trihalogenpyrimidin der allgemeinen Formel (VI), 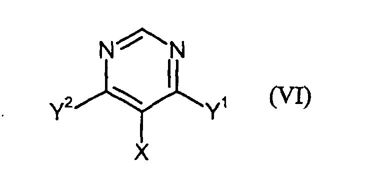 in welcher
X, Y¹ und Y² gleich oder verschieden sind und jeweils für Halogen stehen,
gegebenenfalls in Gegenwart eines Verdünnungsmittels, gegebenenfalls in Gegenwart eines Säureakzeptors und gegebenenfalls in Gegenwart eines Katalysators, umsetzt.

12. Verfahren gemäß Anspruch 10, **dadurch gekennzeichnet, dass** man zur Herstellung von Verbindungen der Formel (I) pro Mol des 2-(2-Hydroxy-phenyl)-2-methoximinoacetonitril der Formel (II) 0,5 bis 15 mol substituiertes Halogenpyrimidin der Formel (III) einsetzt.

13. Verfahren gemäß Anspruch 10, **dadurch gekennzeichnet, dass** man zur Herstellung von Verbindungen der Formel (I) pro Mol des Phenoxypyrimidins der Formel (IV) 0,5 bis 15 mol einer Ringverbindung der Formel (V) einsetzt.

## Claims

1. Halogenopyrimidines of the general formula (I) 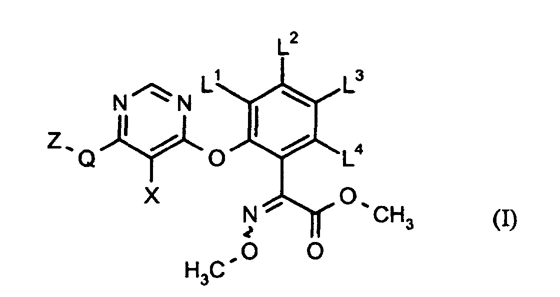 in which
Z represents cycloalkyl having 3 to 7 carbon atoms which is in each case optionally mono- or disubstituted by halogen, alkyl or hydroxyl;
represents heterocyclyl having 3 to 7 ring members which is optionally substituted by alkyl having 1 to 4 carbon atoms or halogen;
or represents phenyl or naphthyl, each of which is optionally mono- to tetrasubstituted by identical or different substituents, where the possible substituents are preferably selected from the list below: halogen, cyano, nitro, amino, hydroxyl, formyl, carboxyl, carbamoyl, thiocarbamoyl;
in each case straight-chain or branched alkyl, hydroxyalkyl, oxoalkyl, alkoxy, alkoxyalkyl, alkylthioalkyl, dialkoxyalkyl, alkylthio, mercaptoalkyl, alkylsulphinyl or alkylsulphonyl having in each case 1 to 8 carbon atoms;
in each case straight-chain or branched alkenyl or alkenyloxy having in each case 2 to 6 carbon atoms;
in each case straight-chain or branched halogenoalkyl, halogeno-alkoxy, halogenoalkylthio, halogenoalkylsulphinyl or halogenoalkylsulphonyl having in each case 1 to 6 carbon atoms and 1 to 13 identical or different halogen atoms;
in each case straight-chain or branched halogenoalkenyl or halogenoalkenyloxy having in each case 2 to 6 carbon atoms and 1 to 11 identical or different halogen atoms;
in each case straight-chain or branched alkylamino, dialkylamino, alkylcarbonyl, alkylcarbonyloxy, alkoxycarbonyl, alkylaminocarbonyl, dialkylaminocarbonyl, arylalkylaminocarbonyl, dialkylaminocarbonyloxy, alkenylcarbonyl or alkinylcarbonyl, having 1 to 6 carbon atoms in the respective hydrocarbon chains;
cycloalkyl or cycloalkyloxy having in each case 3 to 6 carbon atoms;
in each case doubly attached alkylene having 3 or 4 carbon atoms, oxyalkylene having 2 or 3 carbon atoms or dioxyalkylene having 1 or 2 carbon atoms, each of which is optionally mono- to
tetrasubstituted by identical or different substituents from the group consisting of fluorine, chlorine, oxo, methyl, trifluoromethyl and ethyl;
or a grouping 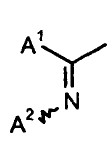 in which
A¹ represents hydrogen, hydroxyl or alkyl having 1 to 4 carbon atoms or cycloalkyl having 1 to 6 carbon atoms and
A² represents hydroxyl, amino, methylamino, phenyl, benzyl or represents in each case optionally cyano-, hydroxyl-, alkoxy-, alkylthio-, alkylamino-, dialkylamino- or phenyl-substituted alkyl or alkoxy having 1 to 4 carbon atoms, or represents alkenyloxy or alkinyloxy having in each case 2 to 4 carbon atoms,
and phenyl, phenoxy, phenylthio, benzoyl, benzoylethenyl, cinnamoyl, heterocyclyl or phenylalkyl, phenylalkyloxy, phenylalkylthio or heterocyclylalkyl having in each case 1 to 3 carbon atoms in the respective alkyl moieties and being in each case optionally mono- to trisubstituted in the ring moiety by halogen and/or straight-chain or branched alkyl or alkoxy having 1 to 4 carbon atoms,
Q represents oxygen or sulphur,
X represents fluorine, chlorine or bromine and
L¹, L², L³ and L⁴ are identical or different and independently of one another each represents hydrogen, halogen, cyano, nitro, or represents alkyl, alkoxy, alkylthio, alkylsulphinyl or alkylsulphonyl having in each case 1 to 6 carbon atoms and being in each case optionally substituted by 1 to 5 halogen atoms.

2. Compounds of the formula (I) according to Claim 1, **characterized in that**
Z represents cyclopentyl or cyclohexyl, each of which is optionally mono- or disubstituted by fluorine, chlorine, methyl, ethyl or hydroxyl;
represents thienyl, pyridyl or furyl, each of which is optionally substituted by methyl, ethyl or chlorine;
or represents phenyl or naphthyl, each of which is optionally mono- to tetrasubstituted by identical or different substituents, where the possible substituents are preferably selected from the list below:
fluorine, chlorine, bromine, iodine, cyano, nitro, amino, hydroxyl, formyl, carboxyl, carbamoyl, thiocarbamoyl,
methyl, ethyl, n- or i-propyl, n-, i-, s- or t-butyl, 1-, 2-, 3-neo-pentyl, 1-, 2-, 3-, 4-(2-methylbutyl), 1-, 2-, 3-hexyl, 1-, 2-, 3-, 4-, 5-(2-methylpentyl), 1-, 2-, 3-(3-methylpentyl), 2-ethylbutyl, 1-, 3-, 4-(2,2-dimethylbutyl), 1-, 2-(2,3-dimethylbutyl), hydroxymethyl, hydroxyethyl, 3-oxobutyl, methoxymethyl, dimethoxymethyl,
methoxy, ethoxy, n- or i-propoxy, methoxymethyl, ethoxymethyl,
mercaptomethyl, methylthio, ethylthio, n- or i-propylthio, methylsulphinyl, ethylsulphinyl, methylsulphonyl or ethylsulphonyl, methylthiomethyl, ethylthiomethyl,
vinyl, allyl, 2-methylallyl, propen-1-yl, crotonyl, propargyl, vinyloxy, allyloxy, 2-methylallyloxy, propen-1-yloxy, crotonyloxy, propargyloxy;
trifluoromethyl, trifluoroethyl,
difluoromethoxy, trifluoromethoxy, difluorochloromethoxy, trifluoroethoxy, difluoromethylthio, trifluoromethylthio, difluorochloromethylthio, trifluoromethylsulphinyl or trifluoromethylsulphonyl,
methylamino, ethylamino, n- or i-propylamino, dimethylamino, diethylamino,
acetyl, propionyl, methoxycarbonyl, ethoxycarbonyl, methylaminocarbonyl, ethylaminocarbonyl, dimethylaminocarbonyl, diethylaminocarbonyl, dimethylaminocarbonyloxy, diethylaminocarbonyloxy, benzylaminocarbonyl, acryloyl, propioloyl,
cyclopentyl, cyclohexyl,
in each case doubly attached propanediyl, ethyleneoxy, methylenedioxy, ethylenedioxy, each of which is optionally mono- to tetrasubstituted by identical or different substituents from the group consisting of fluorine, chlorine, oxo, methyl and trifluoromethyl
or a grouping 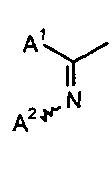 where
A¹ represents hydrogen, methyl or hydroxyl and
A² represents hydroxyl, methoxy, ethoxy, amino, methylamino, phenyl, benzyl or hydroxyethyl, and
phenyl, phenoxy, phenylthio, benzoyl, benzoylethenyl, cinnamoyl, benzyl, phenylethyl, phenylpropyl, benzyloxy, benzylthio, 5,6-dihydro-1,4,2-dioxazin-3-ylmethyl, triazolylmethyl, benzoxazol-2-ylmethyl, 1,3-dioxan-2-yl, benzimidazol-2-yl, dioxol-2-yl, oxadiazolyl, each of which is optionally mono- to tetrasubstituted in the ring moiety by halogen and/or straight-chain or branched alkyl or alkoxy having 1 to 4 carbon atoms,
Q represents oxygen,
X represents fluorine and
L¹, L², L³ and L⁴ are identical or different and independently of one another each represents hydrogen, methyl, ethyl, n- or i-propyl, n-, i-, s- or t-butyl.

3. Compounds of the formula (I) according to Claim 1 in which Q represents oxygen.

4. Compounds of the general formula (IV) 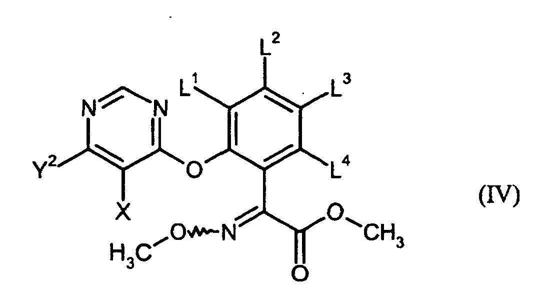 in which
X, L¹, L², L³ and L⁴ are each as defined in Claim 1 and
Y² represents halogen.

5. Compositions, comprising extenders and/or carriers and, if appropriate, surfactants, **characterized in that** they comprise at least one compound as defined in Claims 1 to 3.

6. Method for controlling pests, **characterized in that** compounds as defined in Claims 1 to 3 or compositions as defined in Claim 5 are allowed to act on pests and/or their habitat.

7. Process for preparing pesticides, **characterized in that** compounds of the formula (I) according to Claims 1 to 3 are mixed with extenders and/or surfactants.

8. Use of compounds as defined in Claims 1 to 3 or of compositions as defined in Claim 5 for controlling pests.

9. Use of compounds of the formula (IV) as intermediates.

10. Process for preparing compounds of the general formula (I) according to Claim 1, **characterized in that**
a) 2-(2-hydroxy-phenyl)-2-methoxyiminoacetates of the formula (II) 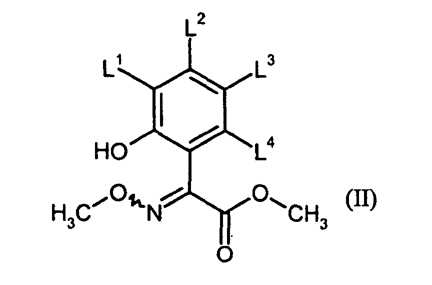 in which
L¹, L², L³ and L⁴ are each as defined in Claim 1
are reacted with a substituted halogenopyrimidine of the general formula (III) 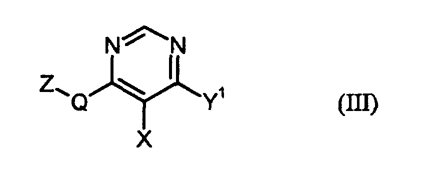 in which
Z, Q and X are each as defined in Claim 1 and
Y¹ represents halogen,
if appropriate in the presence of a diluent, if appropriate in the presence of an acid acceptor and if appropriate in the presence of a catalyst, or
b) phenoxypyrimidines of the general formula (IV) 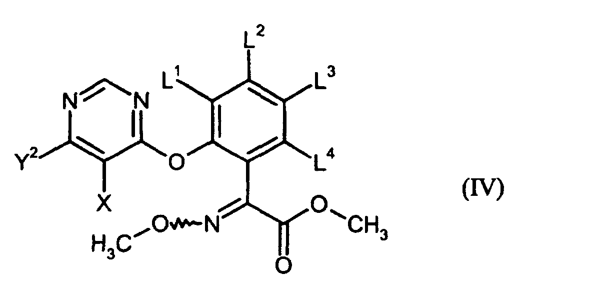
in which
X, L¹, L², L³ and L⁴ are each as defined in Claim 1 and
Y² represents halogen,
are reacted with a cyclic compound of the general formula (V)
Z-Q-H (V)
in which
Z and Q are each as defined above,
if appropriate in the presence of a diluent, if appropriate in the presence of an acid acceptor and if appropriate in the presence of a catalyst.

11. Process for preparing compounds of the formula (IV) according to Claim 4, **characterized in that** 2-(2-hydroxy-phenyl)-2-methoxyimino-acetates of the formula (II) 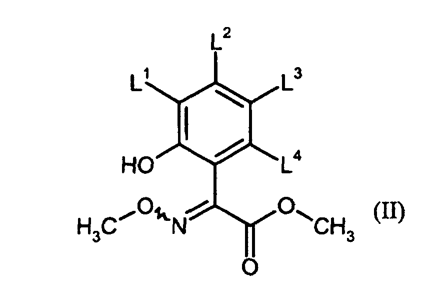 in which
L¹, L², L³ and L⁴ are each as defined in Claim 1
are reacted with a trihalogenopyrimidine of the general formula (VI) 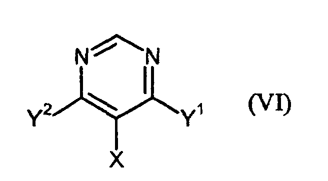 in which
X, Y¹ and Y² are identical or different and each represents halogen,
if appropriate in the presence of a diluent, if appropriate in the presence of an acid acceptor and if appropriate in the presence of a catalyst.

12. Process according to Claim 10, **characterized in that**, to prepare compounds of the formula (I), from 0.5 to 15 mol of substituted halogenopyrimidine of the formula (III) are employed per mole of the 2-(2-hydroxy-phenyl)-2-methoxyiminoacetate of the formula (II).

13. Process according to Claim 10, **characterized in that**, to prepare compounds of the formula (I), from 0.5 to 15 mol of a cyclic compound of the formula (V) are employed per mole of the phenoxypyrimidine of the formula (IV).

## Revendications

1. Halogénopyrimidines de la formule générale (I) : 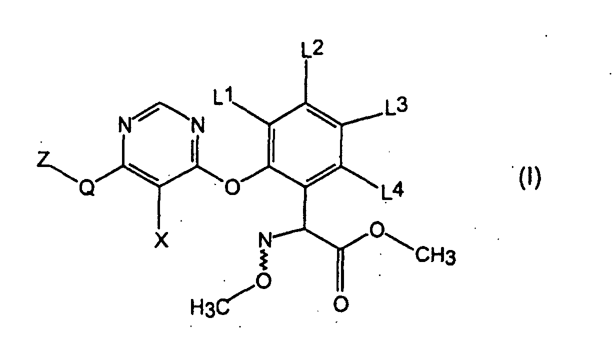 dans laquelle :
Z représente un radical cycloalcoyle ayant 3 à 7 atomes de carbone, le cas échéant substitué une à deux fois par un atome d'halogène, un radical alcoyle
ou le radical hydroxyle ;
représente un radical hétérocyclyle ayant 3 à 7 atomes cycliques, le cas échéant substitué par un radical alcoyle ayant 1 à 4 atomes de carbone ou un atome d'halogène, ou
représente le radical phényle ou naphtyle, le cas échéant substitué une à quatre fois, de manière identique ou différente, où les substituants possibles sont choisis de préférence dans la liste suivante :
un atome d'halogène, le radical cyano, nitro, amino, hydroxyle, formyle, carboxy, carbamoyle, thiocarbamoyle ;
un radical alcoyle, hydroxyalcoyle, oxoalcoyle, alcoxy, alcoxyalcoyle, alcoylthioalcoyle, dialcoxyalcoyle, alcoylthio, mercaptoalcoyle, alcoylsulfinyle ou alcoylsulfonyle, chaque fois linéaire ou ramifié, ayant chaque fois 1 à 8 atomes de carbone ;
un radical alcényle ou alcényloxy, linéaire ou ramifié, ayant chaque fois 2 à 6 atomes de carbone ;
un radical halogénoalcoyle, halogénoalcoxy, halogénoalcoylthio, halogénoalcoylsulfinyle ou halogénoalcoylsulfonyle, chaque fois linéaire ou ramifié, ayant chaque fois 1 à 6 atomes de carbone et 1 à 13 atomes d'halogène identiques ou différents ;
un radical halogénoalcényle ou halogénoalcényloxy, linéaire ou ramifié, ayant chaque fois 2 à 6 atomes de carbone et 1 à 11 atomes d'halogène identiques ou différents ;
un radical alcoylamino ou dialcoylamino, linéaire ou ramifié ;
un radical alcoylcarbonyle, alcoylcarbonyloxy, alcoxycarbonyle, alcoylaminocarbonyle, dialcoylaminocarbonyle, arylalcoylaminocarbonyle, dialcoylaminocarbonyloxy, alcénylcarbonyle ou alcynylcarbonyle, ayant 1 à 6 atomes de carbone dans chaque chaine hydrocarbure ;
un radical cycloalcoyle ou cycloalcoyloxy, ayant chaque fois 3 à 6 atomes de carbone ;
un radical alcoylène ayant 3 ou 4 atomes de carbone, oxyalcoylène ayant 2 ou 3 atomes de carbone ou dioxyalcoylène ayant 1 ou 2 atomes de carbone, chaque fois relié deux fois, le cas échéant substitué une à quatre fois, de manière identique ou différente, par l'atome de fluor, de chlore, le radical oxo, méthyle, trifluorométhyle ou éthyle ;
ou un groupement : 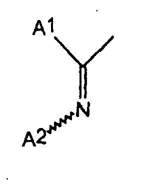 où
A¹ représente l'atome d'hydrogène, le radical hydroxyle ou un radical alcoyle ayant 1 à 4 atomes de carbone ou cycloalcoyle ayant 1 à 6 atomes de carbone, et
A² représente le radical hydroxyle, amino, méthylamino, phényle, benzyle ou un radical alcoyle ou alcoxy ayant 1 à 4 atomes de carbone, chaque fois le cas échéant substitué par le radical cyano, hydroxyle, alcoxy, alcoylthio, alcoylamino, dialcoylamino ou phényle, ou représente un radical alcényloxy ou alcynyloxy ayant chaque fois, 2 à 4 atomes de carbone ;
ainsi qu'un radical phényle, phénoxy, phénylthio, benzoyle, benzoyléthényle, cinnamoyle, hétérocyclyle ou phénylalcoyle, phénylalcoyloxy, phénylalcoylthio ou hétérocyclylalcoyle, ayant chaque fois 1 à 3 atomes de carbone dans chaque chaine alcoyle, le cas échéant substitué dans le partie cyclique, une à trois fois, par un atome d'halogène et/ou un radical alcoyle ou alcoxy linéaire ou ramifié, ayant 1 à 4 atomes de carbone ;
Q représente l'atome d'oxygène ou de soufre,
X représente l'atome de fluor, de chlore ou de brome, et
L¹, L², L³ et L⁴ sont identiques ou différents et représentent indépendamment l'un de l'autre, l'atome d'hydrogène, un atome d'halogène, le radical nitro, cyano, un radical alcoyle, alcoxy, alcoylthio, alcoylsulfinyle ou alcoylsulfonyle ayant 1 à 6 atomes de carbone, chaque fois le cas échéant substitué par à 1 à 5 atomes d'halogène.

2. Composés de la formule (I) selon la revendication 1, **caractérisés en ce que** :
Z représente le radical cyclopentyle ou cyclohexyle, le cas échéant substitué une à deux fois par l'atome de fluor, de chlore, le radical méthyle, éthyle ou hydroxyle ;
représente le radical thiényle, pyridyle ou furyle, le cas échéant substitué par le radical méthyle, éthyle ou l'atome de chlore, ou
représente le radical phényle ou naphtyle, le cas échéant substitué une à quatre fois, de manière identique ou différente, où les substituants possibles sont choisis de préférence dans la liste suivante :
l'atome de fluor, de chlore, de brome, d'iode, le radical cyano, nitro, amino, hydroxyle, formyle, carboxy, carbamoyle, thiocarbamoyle ;
le radical méthyle, éthyle, n- ou i-propyle, n-, i-,s- ou t-butyle, 1-, 2-, 3-, néopentyle, 1-, 2-, 3-, 4-(2-méthylbutyle), 1-, 2-, 3-hexyle, 1-, 2-, 3-, 4-, 5-(2-méthylpentyle), 1-, 2-, 3-(3-méthylpentyle), 2-éthylbutyle, 1-, 3-, 4-(2,2-diméthylbutyle), 1-, 2-(2,3-diméthylbutyle), hydroxyméthyle, hydroxyéthyle, 3-oxobutyle, méthoxyméthyle, diméthoxyméthyle ;
le radical méthoxy, éthoxy, n- ou i-propoxy, méthoxyméthyle, éthoxyméthyle, mercaptométhyle, méthylthio, éthylthio, n- ou i-propylthio, méthylsulfinyle, éthylsulfinyle, méthylsulfonyle ou éthylsulfonyle, méthylthiométhyle, éthylthiométhyle,
le radical vinyle, allyle, 2-méthylallyle, propèn-1-yle, crotonyle, propargyle, vinyloxy, allyloxy, 2-méthylallyloxy, propèn-1-yloxy, crotonyloxy, propargyloxy ;
le radical trifluorométhyle, trifluoroéthyle, difluorométhoxy, trifluorométhoxy, difluorochlorométhoxy, trifluoroéthoxy, difluorométhylthio, trifluorométhylthio, difluorochlorométhylthio, trifluorométhylsulfinyle ou trifluorométhylsulfonyle ;
le radical méthylamino, éthylamino, n- ou i-propylamino, diméthylamino, diéthylamino ;
le radical acétyle, propionyle, méthoxycarbonyle, éthoxycarbonyle, méthylaminocarbonyle, éthylaminocarbonyle, diméthylaminocarbonyle, diéthylaminocarbonyle, diméthylaminocarbonyloxy, diéthylaminocarbonyloxy, benzylaminocarbonyle, acryloxy, propioloyle ;
le radical cyclopentyle, cyclohexyle ;
le radical propanediyle, éthylèneoxy, méthylènedioxy, éthylènedioxy, chaque fois relié deux fois, le cas échéant substitué une à quatre fois, de manière identique où différente, par l'atome de fluor, de chlore, le radical oxo, méthyle ou trifluorométhyle ;
ou un groupement : 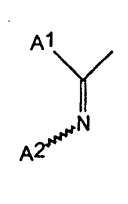 où
A¹ représente l'atome d'hydrogène, le radical méthyle ou hydroxyle, et A² représente le radical hydroxyle, méthoxy, éthoxy, amino, méthylamino, phényle, benzyle ou un hydroxyéthyle ;
ainsi qu'un radical phényle, phénoxy, phénylthio, benzoyle, benzoyléthényle, cinnamoyle, benzyle, phényléthyle, phénylpropyle, benzyloxy, benzylthio, 5,6-dihydro-1,4,2-dioxazin-3-ylméthyle, triazolylméthyle, benzoxazol-2-ylméthyle, 1,3-dioxann-2-yle, benzimidazol-2-yle, dioxol-2-yle, oxadiazolyle, le cas échéant substitué dans la partie cyclique, une à quatre fois, par un atome d'halogène et/ou un radical alcoyle ou alcoxy linéaire ou ramifié, ayant 1 à 4 atomes de carbone ;
Q représente l'atome d'oxygène ;
X représente l'atome de fluor, et
L¹, L², L³ et L⁴ sont identiques ou différents
et représentent indépendamment l'un de l'autre, l'atome d'hydrogène, le radical méthyle, éthyle, n- ou i-propyle, n-, i-, s- ou t-butyle.

3. Composés de la formule (I) selon la revendication 1, dans lesquels Q représente l'atome d'oxygène.

4. Composés de la formule générale (IV) : 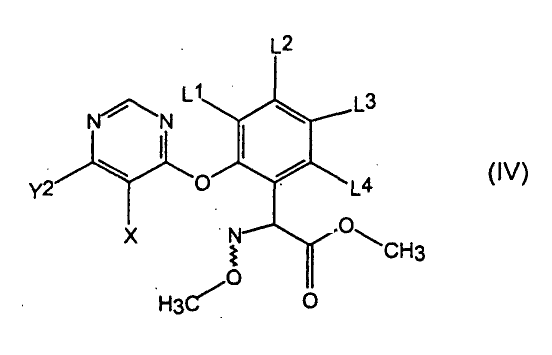 dans laquelle :
X, L¹, L², L³ et L⁴ ont les significations indiquées à la revendication 1, et
Y² représente un atome d'halogène.

5. Agent contenant des diluants et/ou des substances support, ainsi que le cas échéant, des substances tensioactives, **caractérisé par** une teneur en au moins un composé tel que défini aux revendications 1 à 3.

6. Procédé pour lutter contre les parasites, **caractérisé en ce que** l'on fait agir des composés tels que définis aux revendications 1 à 3 ou un agent tel que défini à la revendication 5, sur les parasites et/ou leur biotope.

7. Procédé de préparation d'un agent de lutte contre les parasites, **caractérisés en ce que** l'on mélange des composés de la formule (I) tels que définis aux revendications 1 à 3 avec des diluants et/ou agents tensioactifs.

8. Utilisation des composés tels que définis aux revendications 1 à 3 ou de l'agent tel que défini à la revendication 5, pour lutter contre les parasites.

9. Utilisation des composés de la formule (IV) comme produit intermédiaire.

10. Procédé de préparation de composés de la formule générale (I), selon la revendication 1, **caractérisé en ce que**
a) on fait réagir un ester 2-(2-hydroxyphényl)-2-méthoxyiminoacétique de la formule (II) : 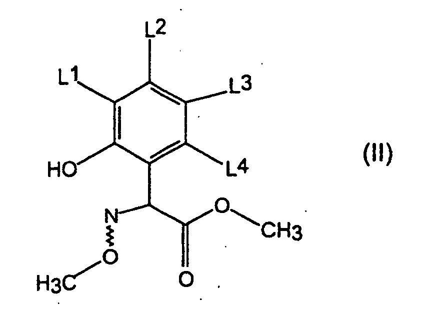 dans laquelle :
L¹, L², L³ et L⁴ ont les significations indiquées à la revendication 1,
avec une halogénopyrimidine substituée de la formule générale (III) : 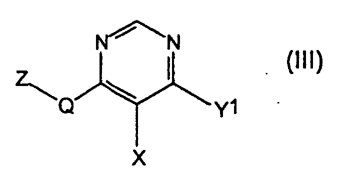
dans laquelle :
Z, Q et X ont les significations indiquées à la revendication 1, et
Y¹ représente un atome d'halogène,
le cas échéant en présence d'un agent de dilution, le cas échéant en présence d'un accepteur d'acide et le cas échéant, en présence d'un catalyseur,
ou
b) on fait réagir une phénoxypyrimidine de la formule générale (IV) : 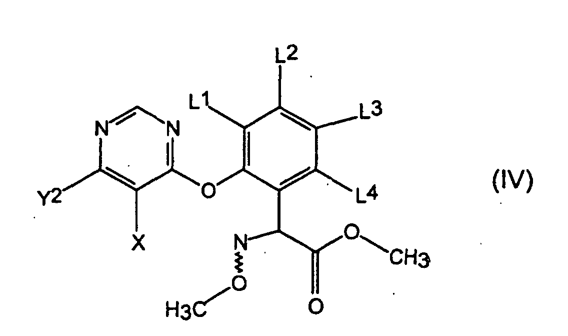
dans laquelle :
X, L¹, L², L³ et L⁴ ont les significations indiquées à la revendication 1, et
Y² représente un atome d'halogène avec un composé cyclique de la formule générale (V) :
Z - Q - H (V)
dans laquelle :
Z et Q ont les significations indiquées ci-dessus,
le cas échéant en présence d'un agent de dilution, le cas échéant en présence d'un accepteur d'acide et le cas échéant, en présence d'un catalyseur.

11. Procédé de préparation de composés de la formule (IV) selon la revendication, **caractérisé en ce que** l'on fait réagir un ester 2-(2-hydroxyphényl)-2-méthoxyiminoacétique de la formule (II) : 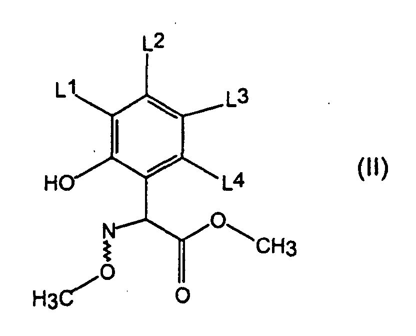 dans laquelle :
L¹, L², L³ et L⁴ ont les significations indiquées à la revendication 1,
avec une trihalogénopyrimidine de la formule générale (VI) : 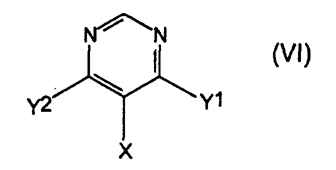
dans laquelle :
X, Y¹ et Y² sont identiques ou différents et représentent un atome d'halogène, le cas échéant en présence d'un agent de dilution, le cas échéant en présence d'un accepteur d'acide et le cas échéant, en présence d'un catalyseur.

12. Procédé suivant la revendication 10, **caractérisé en ce que** l'on met en oeuvre pour la préparation des composés de la formule (I), par mole du 2-(2-hydroxyphényl)-2-méthoximinoacétonitrile de la formule (II), 0,5 à 15 moles d'halogénopyrimidine de la formule (III).

13. Procédé suivant la revendication 10, **caractérisé en ce que** l'on met en oeuvre pour la préparation des composés de la formule (I), par mole de phénoxypyrimidine de la formule (IV), 0,5 à 15 moles d'un composé cyclique de la formule (V).
